# EUROPEAN PATENT SPECIFICATION

(11) **EP 1 766 085 B1**
(45) Date of publication and mention of the grant of the patent: **11.03.2009**
(21) Application number: 05779974.4
(22) Date of filing: 03.05.2005
(51) Int. Cl.: C12Q 1/68, G01N 33/53, C12N 15/11

(54) **APTAMER-NANOPARTICLE CONJUGATES AND METHOD OF USE FOR TARGET ANALYTE DETECTION**
APTAMER-NANOPARTIKELKONJUGATE UND VERWENDUNGSVERFAHREN ZUR DETEKTION VON ZIELANALYTEN
CONJUGUES NANOPARTICULES-APTAMERES, ET LEUR PROCEDE D'UTILISATION POUR DETECTER UN ANALYTE CIBLE

(30) Priority: 03.05.2004 US 567874 P; 22.11.2004 US 995051
(43) Date of publication of application: 28.03.2007
(73) Proprietor: Nanosphere, Inc., Northbrook, Illinois 60062 (US)
(72) Inventor: MÜLLER, Uwe, R., Waukegan, IL 60087 (US); STORHOFF, James, J., Evanston, IL 60202 (US); SENICAL, Michael, J., Chicago, IL 60645 (US); GARIMELLA, Viswanadham, Vernon Hills, IL 60061 (US)
(74) Representative: Grund, Martin
(86) International application number: PCT/US2005/016201
(87) International publication number: WO 2005/113817

(56) References cited:
- WO-A-00/11446
- WO-A-00/70329
- WO-A-96/40991
- WO-A-03/050290
- WO-A-03/062422
- WO-A-03/075765
- WO-A-03/095973
- WO-A-20/04013628
- WO-A-20/05008222
- US-A- 5 843 653
- US-A1- 2002 037 506
- US-A1- 2003 143 580
- US-A1- 2003 162 216
- US-B1- 6 303 316
- US-B1- 6 506 564
- US-B1- 6 680 377
- FAMULOK M ET AL: "APTAMERS AS TOOLS IN MOLECULE BIOLOGY AND IMMUNOLOGY" CURRENT TOPICS IN MICROBIOLOGY AND IMMUNOLOGY, SPRINGER, BERLIN,, DE, vol. 243, 1999, pages 123-136, XP002952168 ISSN: 0070-217X
- POTYRAILO R A ET AL: "ADAPTING SELECTED NUCLEIC ACID LIGANDS (APTAMERS) TO BIOSENSORS" ANALYTICAL CHEMISTRY, AMERICAN CHEMICAL SOCIETY. COLUMBUS, US, vol. 70, no. 16, 15 August 1998 (1998-08-15), pages 3419-3425, XP000784025 ISSN: 0003-2700

## Description

### FIELD OF THE INVENTION

The invention relates to aptamer probes, nanoparticle-aptamer conjugate probes, aptamer arrays, methods of detecting target analytes in a sample comprising detecting binding of a target analyte with aptamer probes, method of detection, and kits.

### BACKGROUND OF THE INVENTION

The detection of protein analytes on antibody microarrays has emerged as a powerful tool for proteomics as well as diagnostics (Macbeath, G.; Schreiber, S. L. Science (2000), 289, 1760-1763; Moody, M. D.; Van Arsdell, S. W.; Murphy, K. P.; Orencole, S. F.; Burns, C. Biotechniques (2001), 31, 186-194; Nielsen, U. B.; Geierstanger, B. H. Journal Immunol. Meth. (2004), 290, 107-120) A variety of different detection methods have been developed for labeling antibody arrays including, but not limited to, fluorescence, (Macbeath, G.; Schreiber, S. L. Science (2000), 289, 1760-1763; Li, Y. L.; Reichert, W. M. Langmuir (2003), 19, 1557-1566) chemiluminescence (Moody, M. D.; Van Arsdell, S. W.; Murphy, K. P.; Orencole, S. F.; Burns, C. Biotechniques 2001, 31, 186-194 ), resonance light scattering (Nielsen, U. B.; Geierstanger, B. H. Journal Immunol. Meth.(2004), 290, 107-120.), and SERS (Grubisha, D. S.; Lipert, R. J.; Park, H.-Y.; Driskell, J.; Porter, M. D. Anal. Chem. (2003), 75, 5936-5943 ). Signal amplification strategies such as rolling circle amplification (RCA) also have been used to increase the detection sensitivity of fluorescence-based strategies. (Schweitzer, B Schweitzer, B.; Roberts, S.; Grimwade, B.; Shao, W.; Wang, M.; Fu, Q.; Shu, Q.; Laroche, I.; Zhou, Z:; Tchernev, V. T.; Christiansen, J.; Velleca, M.; Kingsmore, S. F. Nat. Biotechnol. (2002), 20, 359-365.; Wiltshire, S.; Lambert, J.; O'Malley, S.; Kukanskis, K.; Zhu, Z.; Kingsmore, S. F.; Lizardi, P. M.; Ward, D. C. Proc. Natl. Acad. Sci. U. S. A. (2000), 97, 10113-10119; ) These methods have provided high sensitivity detection (< 10 pg/mL) of protein analytes, but the use of such labeling strategies has been limited by the performance of the antibodies which are prone to cross reactivity (Nielsen, U. B.; Geierstanger, B. H. Journal Immunol. Meth. (2004), 290, 107-120). In addition, the reproducible preparation of highly purified antibody reagents is both challenging and time consuming (Jayasena, S. D. Clin. Chem. (1999), 45, 1628-1650). Accordingly, there is a need in the field for a probe system that provides not only high sensitivity and specificity for the protein analyte of interest, but also reproducibility in production and use.

RNA and DNA aptamers can substitute for monoclonal antibodies in various applications (Jayasena, "Aptamers: an emerging class of molecules that rival antibodies in diagnostics." Clin. Chem., 45(9):1628-50, 1999; Morris et al., "High affinity ligands from in vitro selection: complex targets." Proc. Natl. Acad. Sci., USA, 95(6):2902-7, 1998). Aptamers are nucleic acid molecules having specific binding affinity to non-nucleic acid or nucleic acid molecules through interactions other than classic Watson-Crick base pairing. Aptamers are described, for example, in U.S. Patent Nos. 5,475,096; 5,270,163; 5,589,332; 5,589,332; and 5,741,679.

The relatively fast selection process of the specific aptamers and the inexpensive synthesis makes the aptamer useful alternatives for monoclonal antibodies. These nucleic acids can be easily synthesized, readily manipulated, and can be stored for long periods of time. These benefits make nucleic acids more attractive biotechnology tools than their counterpart of proteins, antibodies. Additionally these nucleic acid probes can also be labeled by radioisotope, biotin, or fluorescent tags and can be used to detect targets under various conditions. An increasing number of DNA and RNA aptamers that recognize their non-nucleic acid targets have been developed by SELEX and have been characterized (Gold et al., "Diversity of Oligonucleotide Functions," Annu. Rev. Biochem., 64:763-97.1995; Bacher & Ellington, "Nucleic Acid Selection as a Tool for Drug Discovery," Drug Discovery Today, 3(6):265-273, 1998). US 2002/0037506 describes an aptamer based two-site binding sandwich assay, employing nucleic acid ligands as capture and/or reporter molecules. US 2003/0162216 discloses a nucleic acid ligand biochip, consisting of a solid support to which one or more specific nucleic acid ligands is attached in a spatially defined manner. Further, WO 96/40991 describes an immunoassay termed as ELONA, employing nucleic acid ligands as capture molecules and/or detector molecules. WO 2004/013628 discloses a fluorescence polarization assay using fluorescence anisotropy. In addition, WO 2005/008222 describes a method for detecting analytes based on evanescent illumination and scatter based detection of nanoparticle probe complexes.

### SUMMARY OF THE INVENTION

The invention provides in its broadest scope:
A method for detecting at least one typ of target analyte in a sample, the target analyte having at least two binding sites, the method comprising the steps of:
   a) providing at least one type of nanoparticle probe comprising detector aptamers, wherein the detector aptamers on each type of probe have a configuration that can bind to a first binding site of a specific type of target analyte;
   b) contacting the sample with the nanoparticle probe under conditions that are effective for the binding of the detector aptamers to the target analyte; and
   c) detecting whether the detector aptamer binds to the target analyte,
      wherein the aptamers are present on the nanoparticles at a surface density ranging from between 8.9 x 10" and 6.4 x 10¹² aptamers/cm².
A nanoparticle-aptamer conjugate probe comprising:
   a) nanoparticles; and
   b) at least one type of aptamer, the aptamers being present on the nanoparticles at a surface density ranging from between 8.9 x 10¹¹ and 6.4 x 10¹² aptamers/cm².

The following subject-matter is also disclosed in the present specification:
Methods of detecting analytes, including non-nucleic acid and nucleic acid molecules. The methods comprise contacting an
analyte with nanoparticles having aptamers attached thereto (nanoparticle-aptamer conjugates), wherein the aptamers have a configuration capable of binding to specific target analytes. The contacting takes place under conditions effective to allow binding of the aptamers on the nanoparticles with the analyte. The binding of the aptamers on the nanoparticles with the analyte results in a detectable change.

A method is provided for detecting at least one target analyte in a sample, the target analyte having at least two binding sites, the method comprising the steps of: a) providing a substrate having at least one type of capture probe bound thereto, wherein the capture probe can bind to a first binding site of a specific target analyte; b) providing at least one type of nanoparticle probe comprising detector aptamers, wherein the detector aptamers can bind to a second binding site of the target analyte; c) contacting the sample with the substrate and the nanoparticle probe under conditions that are effective for the binding of the capture probe to the first binding site of the target analyte and the binding of the nanoparticle probe to the second binding site of the target analyte to form a complex; and d) detecting for the presence or absence of the complex wherein the presence or absence of the complex is indicative of the presence or absence of the specific target analyte in the same.

In one aspect, a sample can be contacted with the detector probe so that an analyte target present in the sample binds with the detector aptamers on the detector probe, and the analyte target bound to the detector probe can then be contacted with the substrate so that the analyte target binds with the capture aptamer on the substrate. Alternatively, a sample can be contacted with the substrate so that an analyte target present in the sample binds with a capture aptamer, and the analyte target bound to the capture aptamer can then be contacted with the detector probe so that the analyte target binds with the detector aptamers on the detector probe. A sample can be contacted simultaneously with the detector probe and the substrate.

The capture probe comprise an antibody or an capture aptamer. The binding sites on the target analyte are epitopes that a specific capture probe binds to.

Two or more types of nanoparticle probes are provided, each type of nanoparticle probes having detector aptamers bound thereto that are capable of binding to a different epitope on the same target analyte or to different target analytes, or both.

A method is provided for detecting a target analyte in a sample, said target analyte having at least two binding sites, the method comprising the steps of: (a) providing a type of nanoparticles having aptamers bound thereto, the aptamers capable of binding to two or more binding sites of the target analyte; (b) contacting the sample, and the nanoparticles having aptamers bound thereto under conditions effective to allow binding between the target analyte and the aptamers bound to nanoparticles bound thereto; and (c) observing a detectable change brought about by the binding of the target analyte with the aptamers bound to the nanoparticles.

A method is provided for detecting a target analyte in a sample, said target analyte having at least two binding sites, the method comprising the steps of: (a) providing at least two types of nanoparticles having aptamers bound thereto, each type of aptamer capable of binding to a different binding site of the target analyte; (b) contacting the sample, and the at least two types of nanoparticles having aptamers bound thereto under conditions effective to allow binding between the target analyte and the aptamers bound to the nanoparticles; and (c) observing a detectable change brought about by the binding of the the aptamers bound to the nanoparticles with the target analytes.

A method is provided for detecting a target analyte in a sample, said target analyte having at least two binding sites, the method comprising the steps of: (a) providing at least one type of nanoparticles having aptamers bound thereto, the aptamer capable of binding to a binding site of the target analyte and at least one type of nanoparticles having antibodies bound thereto, the antibodies capable to binding to a different binding site of the target analyte; (b) contacting the sample, the at least one type of nanoparticles having aptamers bound thereto and the at least one type of nanoparticles having antibodies bound thereto under conditions effective to allow binding between the target analyte and the aptamers and the antibodies bound to the nanoparticles; and (c) observing a detectable change brought about by the binding of the aptamers bound to the nanoparticles and the antibodies bound to the nanoparticles to the target analyte.

The captured target-nanoparticle probe complex is detected by any suitable means including photonic, electronic, acoustic, opto-acoustic, gravity, electro-chemical, electro-optic, mass-spectrometric, enzymatic, chemical, biochemical, or physical means. In one aspect, a suitable detection method includes the use of silver stain to enhance the presence of nanoparticles, detecting light scattered by the nanoparticle; or visual observation using an optical scanner.

The nanoparticles are made of any suitable material. In one aspect, the nanoparticle are made of a noble metal such as of gold or silver.

Any suitable substrate may be used such as a magnetic bead or a planar surfaced substrate. The substrate may be made from any suitable material such as glass, quartz, ceramic, or plastic. In one aspect of this embodiment, the substrate is addressable and a plurality of capture probes, each of which can recognize a different target analyte, are attached to the substrate in an array of spots. In another aspect of this embodiment, each spot of capture probes may be located between two electrodes, the nanoparticles are made of a material that is a conductor of electricity, and step (d) comprises detecting a change in conductivity. The electrodes and the nanoparticles maybe composed of any conducting material such as gold. If desired, the substrate may be contacted with silver stain to produce the change in conductivity.

An aptamer probe is provided. The aptamer probe comprising: an aptamer having an oligonucleotide tail; and a second linker oligonucleotide having a sequence that is complementary to at least a portion of a sequence of the oligonucleotide tail, said second oligonucleotide having an optional label.

In one aspect, the optional label is a detection label that allows detection by photonic, electronic, acoustic, opto-acoustic, gravity, electro-chemical, electro-optic, mass-spectrometric, enzymatic, chemical, biochemical, or physical means. Representative examples include fluorescent, luminescent, phosphorescent, or radioactive detection labels, a quantum dot, a nanoparticle, a dendrimer, a molecular aggregate or a bead, a nanoparticle, and an oligonucleotide having a known sequence. The oligonucleotide is designed to be amplified by physical, chemical or biochemical means such as hybridization cascades or enzymatic means.

In another aspect, the optional label is a particle - oligonucleotide conjugate. The particle conjugate label comprises particles having one or more types of DNA barcodes bound directly or indirectly to the nanoparticles. These "DNA barcodes" are oligonucleotides that serve as a surrogate for the target analyte and provide a means for signal amplification. The DNA barcodes may further be optionally labeled with a detection label, e.g. a fluorephore. The detection label allows for detection by photonic, electronic, acoustic, opto-acoustic, gravity, electro-chemical, electro-optic, mass-spectrometric, enzymatic, chemical, biochemical, or physical means.

The barcodes that are released may be detected by any suitable means including arrayed substrates in a sandwich assay using any suitable detection probe. The particles may be of any suitable size including nanoparticles and microsized particles and may be made of any suitable material such as polymers (e.g., polystyrene), metals (e.g., gold or silver), ceramics, semiconductor material. When the optional label is a particle-oligonucleotide conjugate, the aptamer probe of the invention are particularly useful in biobarcode detection assays such as the one described in U.S. serial number 10/877,750, filed June 25, 2004.

In another aspect, the second oligonucleotide has a sequence of at least two portions, a first portion bound to the oligonucleotide tail and a second portion bound to an oligonucleotide bound to the nanoparticle conjugate.

A nanoparticle-aptamer conjugate probe is provided. The aptamer probe comprises: (a) nanoparticles; and (b) at least one type of aptamer, the aptamers being present on the nanoparticles at a surface density ranging from between about 1.0 x 10¹⁰ and about 1.0 x 10¹³ aptamers/cm², preferably around 8.0 x 10¹¹ and 6.4 x 10¹². In one aspect, the nanoparticles are metallic or semiconductor nanoparticles. In another aspect, the nanoparticles are made of a noble metal such as gold.

In one aspect, the conjugate probe comprises at least two types of aptamers.

In another aspect, the conjugate probe further comprises diluent oligonucleotides attached thereto in addition to the aptamers. The diluent oligonucleotides include polyadenosine oligonucleotides of any suitable length such as poly A10 [SEQ ID NO. 6] or A20 [SEQ ID NO. 7].

An aptamer of the invention can be a thioaptamer that contain phosphorothioate or phosphorodithioate moieties.

Aptamers attached to a substrate can be located between two electrodes, the nanoparticles can be made of a material that is a conductor of electricity, and step (d) in the methods of the invention can comprise detecting a change in conductivity. A plurality of aptamers, each of which can recognize a different target analyte, attached to a substrate in an array of spots and each spot of aptamers is located between two electrodes, the nanoparticles are made of a material that is a conductor of electricity, and step (d) in the methods comprise detecting a change in conductivity. The electrodes can be made, for example, of gold and the nanoparticles are made of gold. Alternatively, a substrate can be contacted with silver stain to produce a change in conductivity.

A substrate for detection of one or more target analytes is provided. The substrate includes a substrate, at least one type of capture aptamers bound to the substrate, each type of capture aptamers binds to a specific target analyte and arranged in an array of discrete spots; and (c) optional electrodes located between the discrete spots.

A method is provided for detecting at least one target analyte in a sample, the target analyte having at least two binding sites, the method comprising the steps of: (a) providing a substrate having at least one type of capture probe bound thereto, wherein the capture probe can bind to a first binding site of a specific target analyte; (b) providing at least one type of detector aptamer probe, wherein the detector aptamers can bind to a second binding site of the target analyte; (c) contacting the sample with the substrate and the probe under conditions that are effective for the binding of the capture probe to the first binding site of the target analyte and the binding of the aptamer probe to the second binding site of the target analyte to form a complex; and (d) observing for a detectable change. The captured target-aptamer probe complex is detected by photonic, electronic, acoustic, opto-acoustic, gravity, electro-chemical, electro-optic, mass-spectrometric, enzymatic, chemical, biochemical, or physical means.

In one aspect, the capture probe comprises an antibody or an capture aptamer. The binding sites are epitopes that a specific capture probe binds to.

In another aspect, two or more types of aptamer probes are provided, each type of probes having detector aptamers bound thereto that are capable of binding to a different epitope on the same target analyte or to different target analytes, or both.

In another aspect, sample is first contacted with the aptamer probe so that a target analyte present in the sample binds to the detector aptamers on the probe, and the target analyte bound to the aptamer probe is then contacted with the substrate so that the target analyte binds to the capture probe on the substrate.

In another aspect, the sample is first contacted with the substrate so that a target analyte present in the sample binds to a capture probe, and the target analyte bound to the capture aptamer is then contacted with the aptamer probe so that the target analyte binds to the detector aptamers on the aptamer probe.

In another aspect, the sample, the aptamer probe and the capture probe on the substrate are contacted simultaneously.

Any suitable substrate may be used and such substrates may be addressable. Representative substrates are described above. A plurality of capture probes, each of which can recognize a different target analyte, may be attached to the substrate in an array of spots. If desired, each spot of capture probes may located between two electrodes, the optional label on the aptamer probe is a nanoparticle made of a material that is a conductor of electricity, and a change in conductivity may be detected. The electrodes may be made of gold and the nanoparticles may be made of gold.

A method for detecting at least one type of target analyte in a sample, the target analyte having at least two binding sites, the method comprising the steps of: (a) providing at least one type of detector aptamer probe, wherein the detector aptamers on each type of probe has a configuration that can bind to a first binding site of a specific type of target analyte; (c) contacting the sample with the aptamer probe under conditions that are effective for the binding of the detector aptamers to the target analytes; and (d) detecting whether the detector aptamer binds to the target analytes.

A method is provided for detecting a target analyte in a sample, said target analyte having at least two binding sites, the method comprising the steps of: (a) providing a type of detector aptamer probes of claim 33, the aptamers capable of binding to two or more binding sites of the target analyte; (b) contacting the sample, and the aptamer probes having aptamers bound thereto under conditions effective to allow binding between the target analyte and the aptamers ; and (c) observing a detectable change brought about by the binding of the target analyte with the aptamers.

A method for detecting a target analyte in a sample is provided, said target analyte having at least two binding sites, the method comprising the steps of: (a) providing at least two types of aptamer probes of claim 33, each type of aptamer capable of binding to a different binding site of the target analyte; (b) contacting the sample, and the at least two types of aptamers probes under conditions effective to allow binding between the target analyte and the aptamers bound to the nanoparticles; and (c) observing a detectable change brought about by the binding of the the aptamers to the target analyte.

A kit for detecting for one or more analytes in a sample, the kit comprising an aptamer detection probe and an optional substrate. The substrate may be arrayed with at least one capture probe for a specific target analyte.

### BRIEF DESCRIPTION OF THE DRAWINGS

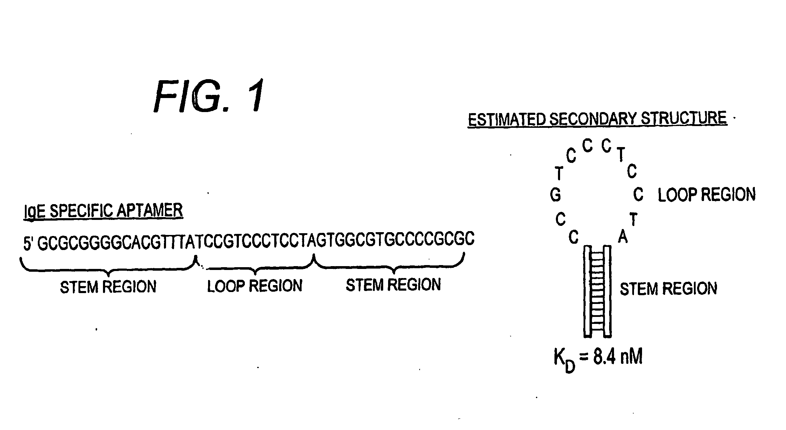

Figure 1 shows an aptamer sequence used for testing. Estimated secondary structure and measured Kd value from previous studies are shown.

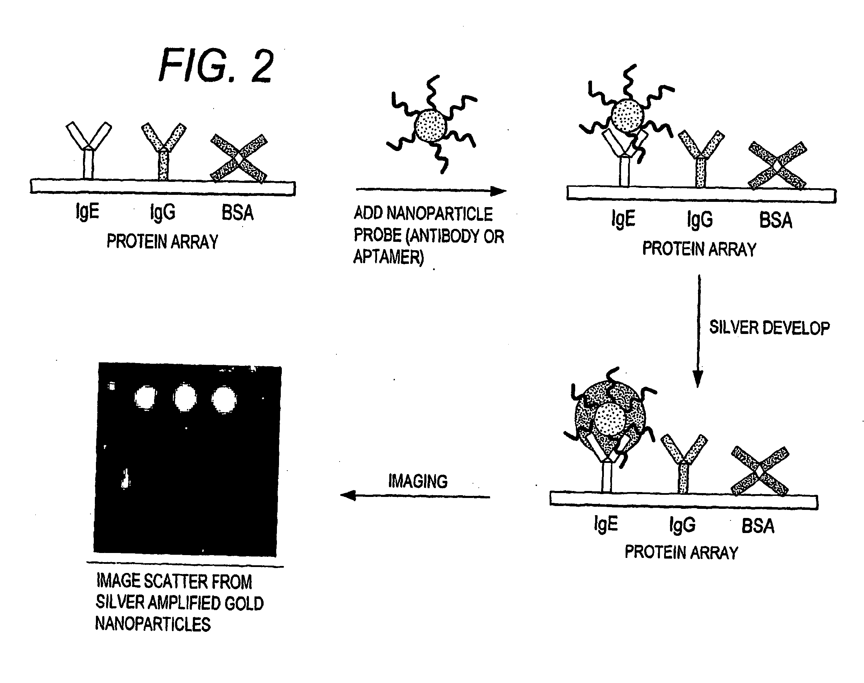

Figure 2 shows a schematic illustration of assay used to demonstrate feasibility of aptamer coated gold probes.

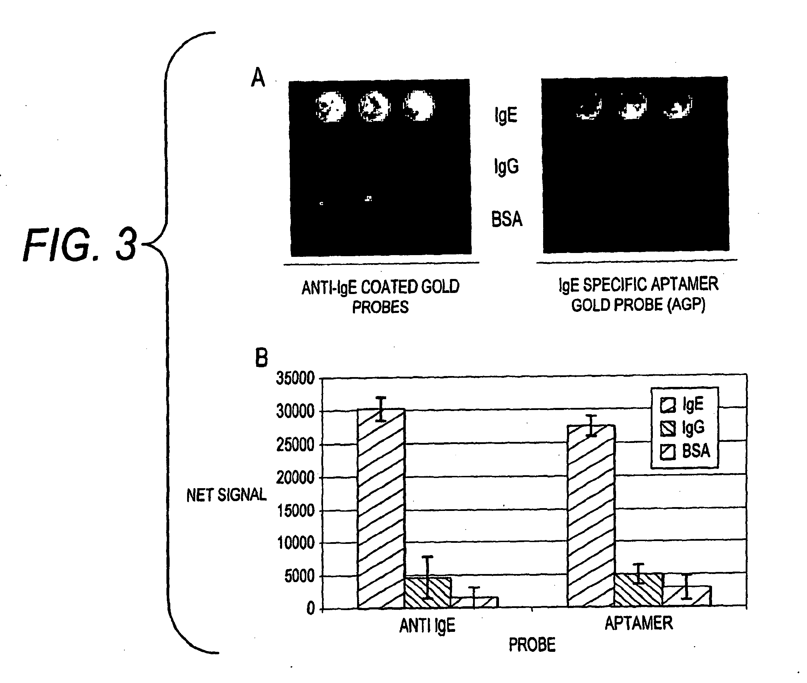

Figure 3 shows a binding comparison of antibody coated gold probes (Anti_IgE) and aptamer coated gold probes to IgE immobilized on glass slides. The probes were exposed to the slides in separate reaction wells followed by silver amplification. A) Image of the glass slides after silver development captured with the Arrayworx image analyzer. B) The net signal intensity from each set of spots averaged over three replicates. Error bar represents one standard deviation.

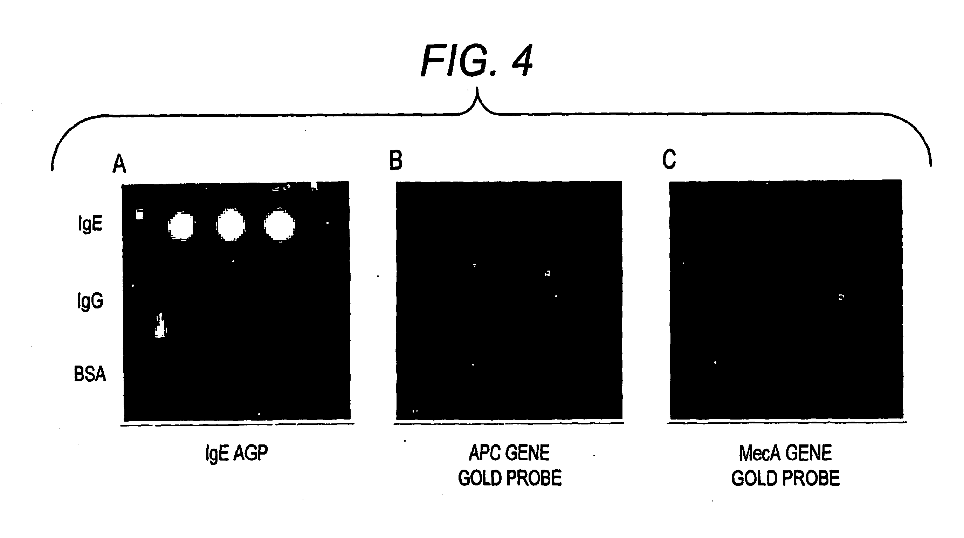

Figure 4 shows the detection specificity of aptamer coated gold probes incubated on protein arrays containing IgE. Detection procedure is shown in Figure 2. A) Protein array incubated with IgE specific aptamer coated gold probes. B) Protein array incubated with DNA-modified particles containing an APC gene sequence. C) Protein array incubated with DNA-modified particles containing a mecA gene sequence.

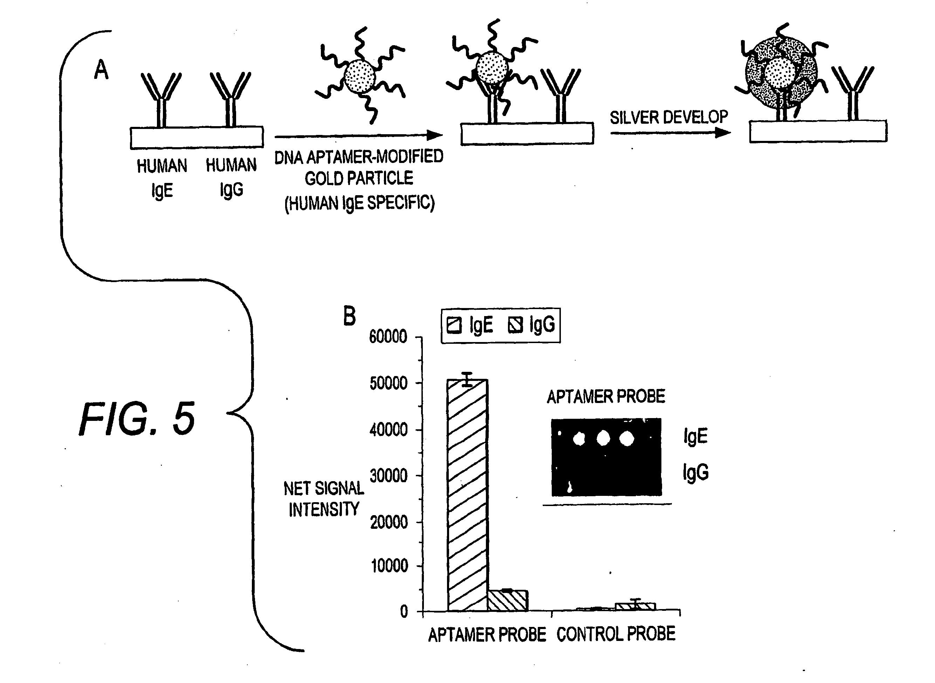

Figure 5 is a schematic illustrating (A) the binding of aptamer coated gold probes to antibody arrays. Silver is deposited onto the aptamer coated gold particles to amplify the signal and (B) binding of anti-human IgE aptamer coated gold probes (AGPs) or control DNA-modified gold probes to human IgE and IgG antibodies immobilized on an array. The net signal intensity from the immobilized antibodies is plotted for each probe. The error bars represent the standard deviation from three replicate spots. The inset shows an image of the array labeled with the aptamer probe after silver development.

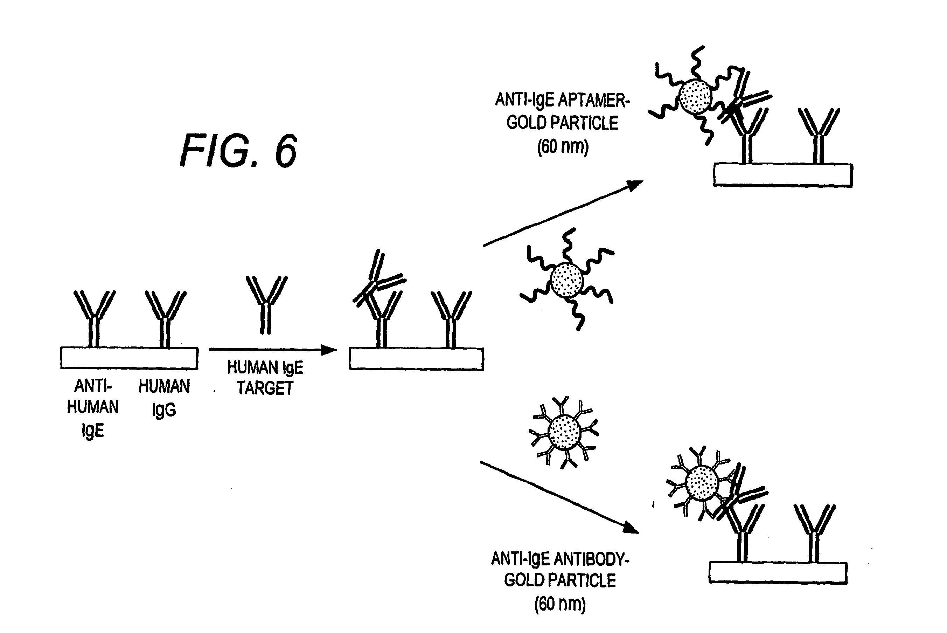

Figure 6 is a schematic illustrating the comparison of aptamer- and antibody-modified 60 nm diameter gold particles as detection labels for human IgE target. The comparison was performed in a sandwich assay format using anti-human IgE antibodies immobilized on glass slides to capture the IgE target. Human IgG antibodies were immobilized on the glass slides as a negative control.

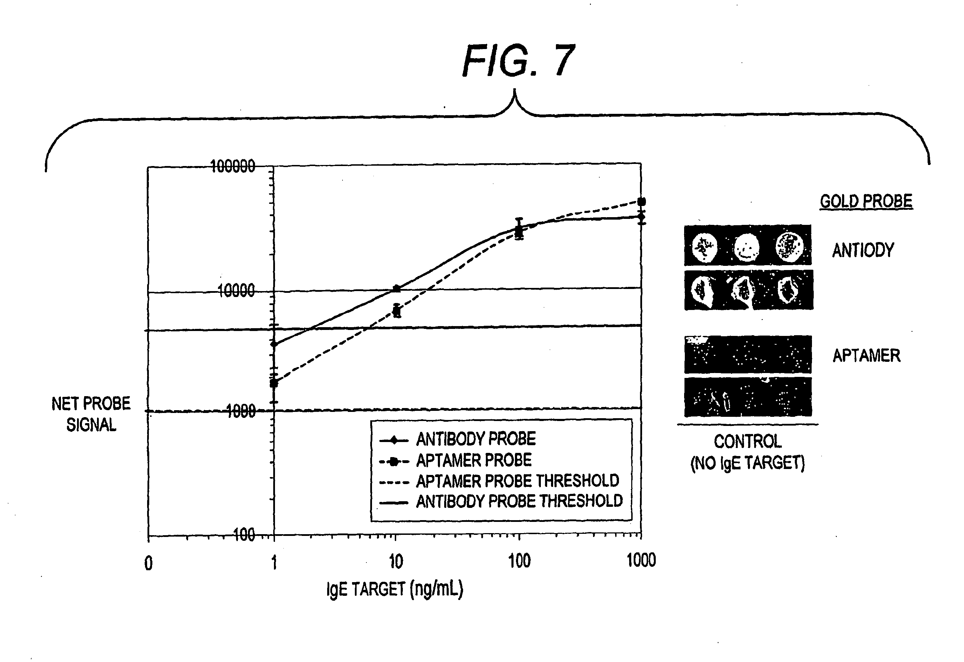

Figure 7 is a schematic illustrating the comparison of human IgE detection using anti-IgE aptamer or antibody coated gold probes. The net signal intensity (signal from IgE target - no target control well) from anti-IgE capture antibody sites on the array is plotted as a function of IgE target concentration for each probe. The error bars represent the standard deviation from the three replicate spots on the array. The threshold line for each probe is three standard deviations above the no target control well. The inset shows images of the goat anti-IgE (top row) and human IgG (bottom row) test sites on the array following incubation of 1 ug/mL human IgG (no IgE target) and subsequent labeling with antibody or aptamer probes.

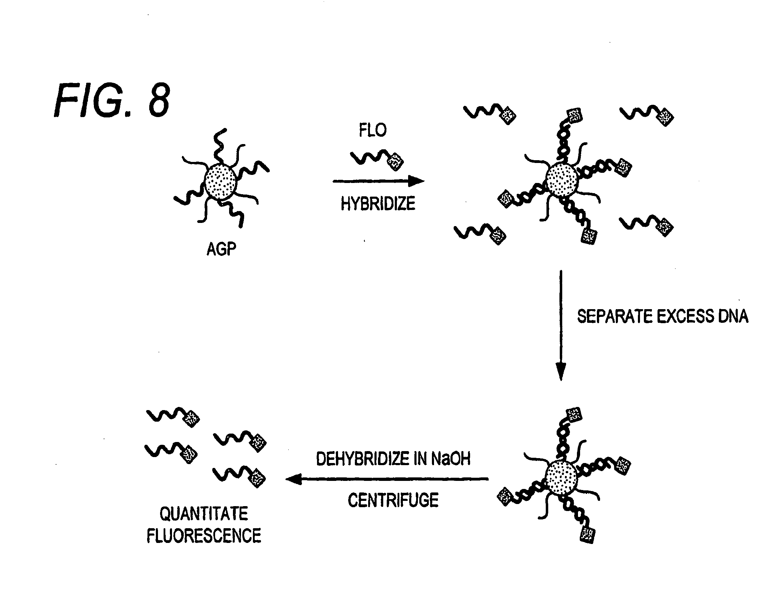

Figure 8 illustrates quantitation of functional aptamer density on gold nanoparticle surfaces through hybridization of fluorophore labeled oligonucleotides (FLOs). The scheme is adapted from Demers et. al Anal. Chem 2000. FLOs are hybridized to the aptamers attached to the gold nanoparticle. The excess FLOs are then removed by centrifugation. The FLOs hybridized to the particle are then released, and the amount of fluorescence is quantitated using a fluorescence plate reader.

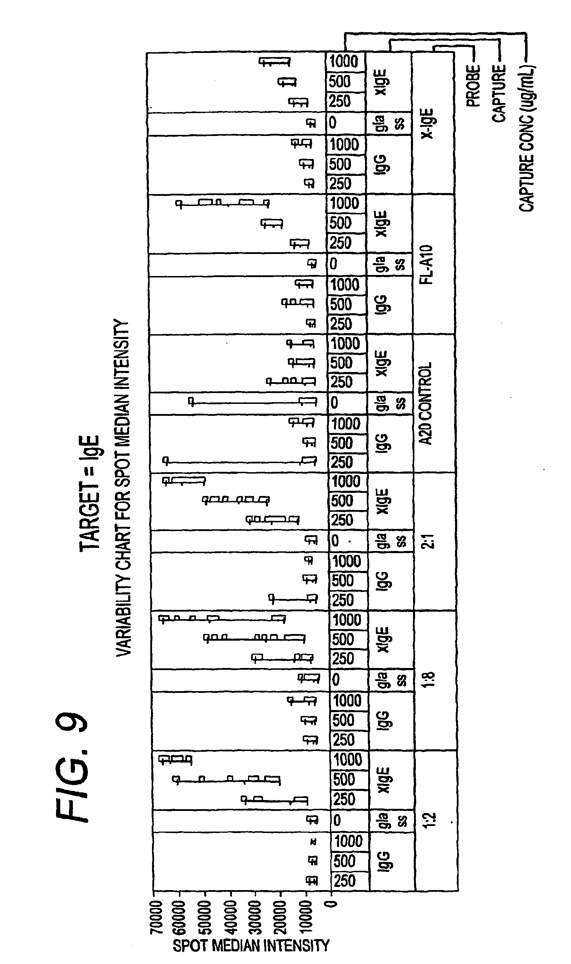

Figure 9 is a plot of spot intensity on an array for designated capture sequences (middle row, x axis). The arrays were incubated with human IgE target, followed by aptamer coated gold probes coated with different aptamer densities, the type of aptamer coated gold probe used to label the array is designated on the bottow row of the x axis based on A10-aptamer 1:A20 ratio. Note that an A20 control probe and anti (x)-IgE probe also were tested as negative and positive control probes. The middle row describes the capture antibody on the slide (IgG or x-IgE), note that a background outside of the spots (glass) also was measured. The top row designates the concentration of capture antibody arrayed onto the slide. The median total scatter intensity (y axis) was measured for each of the capture antibody concentrations and probes tested. Multiple spots on the array were analyzed for each probe.

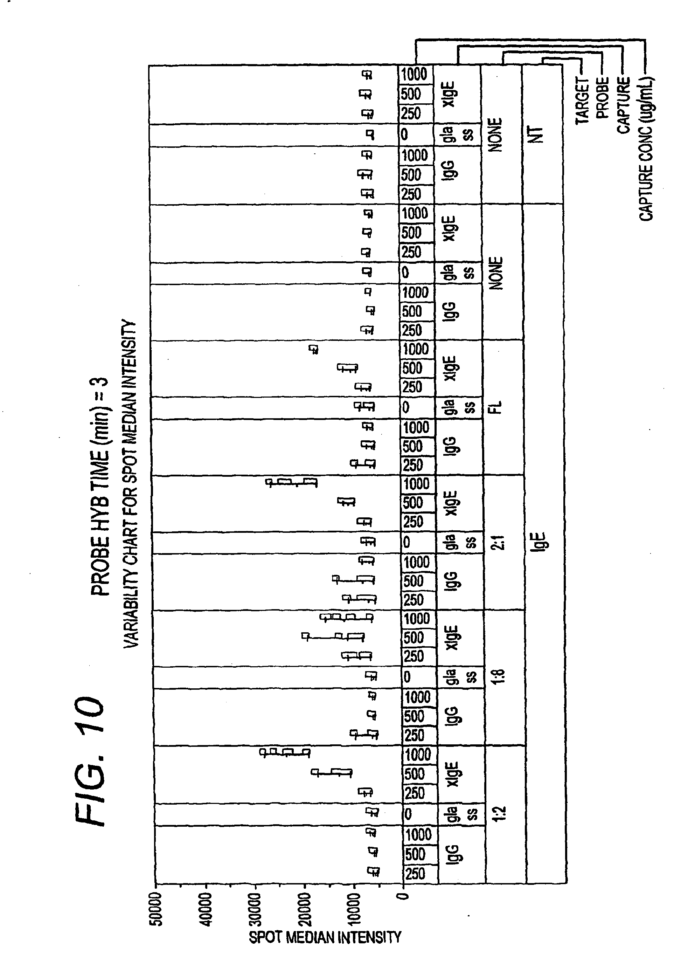

Figure 10 is a plot of spot intensity on an array for designated capture sequences (middle row, x axis). The arrays were incubated with human IgE target, followed by aptamer coated gold probes coated with different aptamer densities. the type of aptamer coated gold probe used to label the array is designated on the bottow row of the x axis. 60 nm diameter gold probes with different aptamer densities were incubated on the array for 3 minutes. The scatter intensity is plotted as a function of the array spot (IgE target or IgG control at 250, 500, or 1000 ug/mL) and the aptamer coated gold probe (1:2, 1:8, 2:1, or FL) used for labeling.

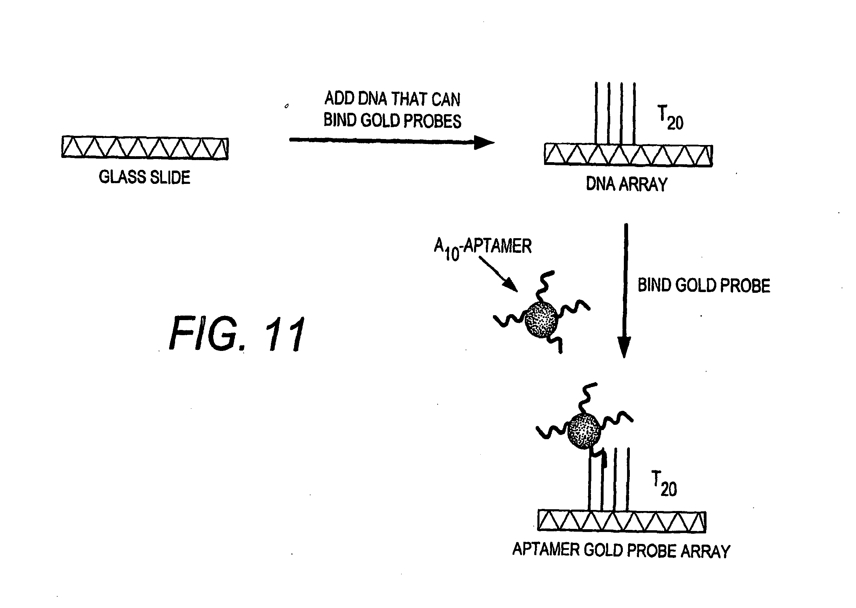

Figure 11 is a schematic illustrating the preparation of aptamer coated gold probe arrays on a glass surface. Step one: DNA is immobilized onto a glass surface. A T₂₀ oligonucleotide is used in this example. Step two: An A₁₀ - anti-IgE aptamer coated gold probe is hybridized to the DNA array.

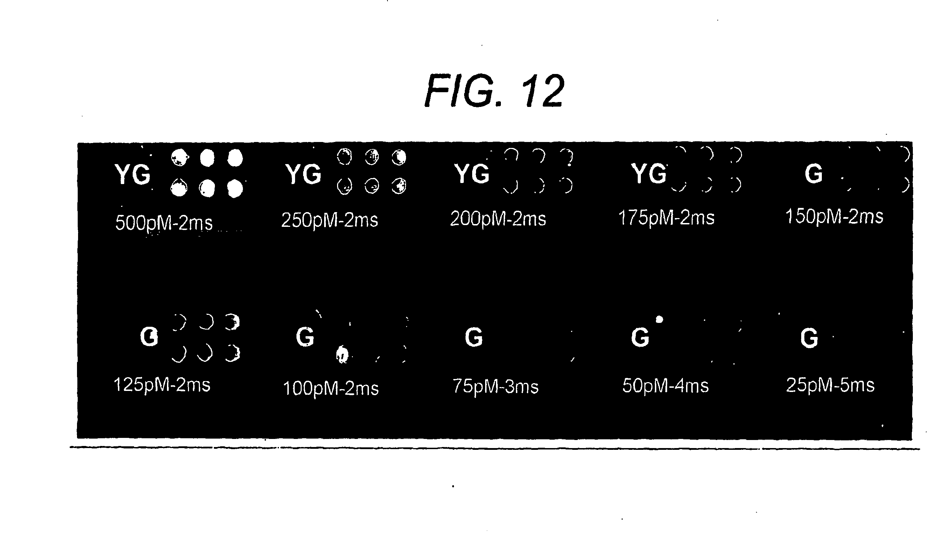

Figure 12 illustrates color images of gold probe arrays prepared with different concentrations of A10-aptamer coated gold particle. The probe arrays were prepared by hybridizing A10-aptamer coated gold particle (50 nm diameter) to a T₂₀ DNA array. Planar illumination of the glass slide with white light generates evanescent induced light scatter from the gold probes. The color images were recorded with a Zeiss Axioplan microscope equipped with a color CCD camera. The probe concentration and exposure time is listed under the image. It should be noted that the scatter color is also detectable visually with the naked eye. Scatter colors are abbreviated as follows: yg = yellow-green, and g = green

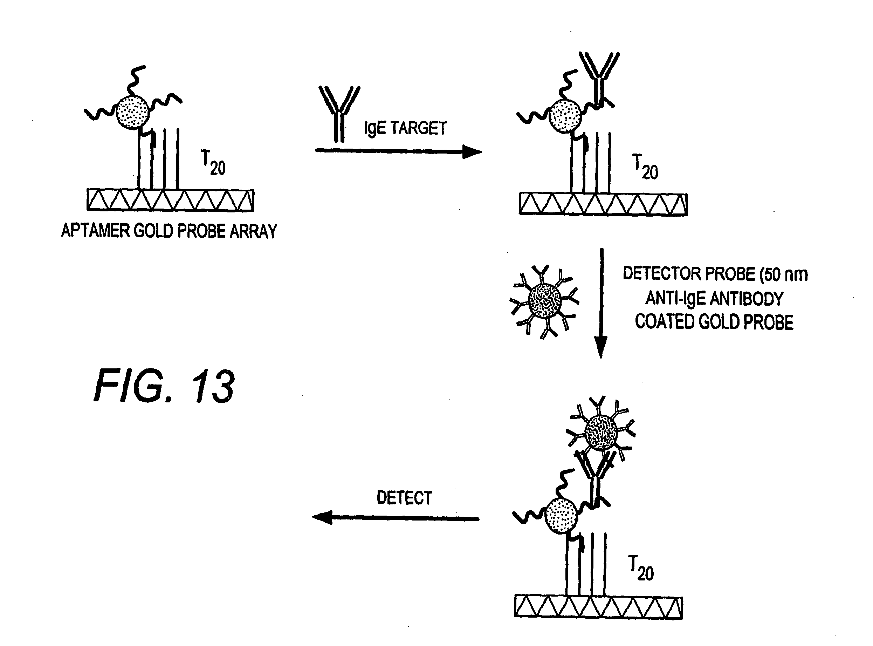

Figure 13 is a schematic illustrating human IgE detection via colorimetric scatter using anti-IgE aptamer coated gold probes. The human IgE target is incubated on the probe array, followed by a detector probe (polyclonal anti-IgE coated 50 nm gold probe) which binds to the human IgE. For detection, the substrate is illuminated with light generating optical scatter from the gold probes, which is monitored with a photosensor.

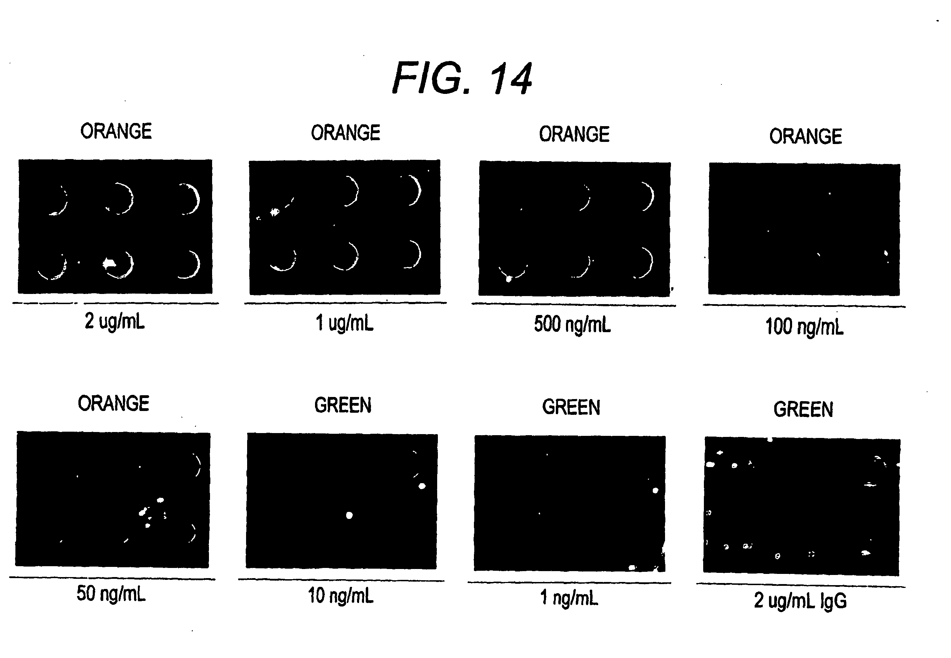

Figure 14 illustrates color images of human IgE assays performed on anti-IgE aptamer coated gold probe arrays. The probe arrays were incubated with different concentrations of human IgE target or an human IgG negative control samples (the target concentration is listed below the image) followed by a detector probe (polyclonal anti-IgE coated 50 nm gold probe). Planar illumination of the glass slide with white light generates evanescent induced light scatter from the gold probes. The color images were recorded with a Zeiss Axioplan microscope equipped with a color CCD camera. The observed scatter color is noted above the image.

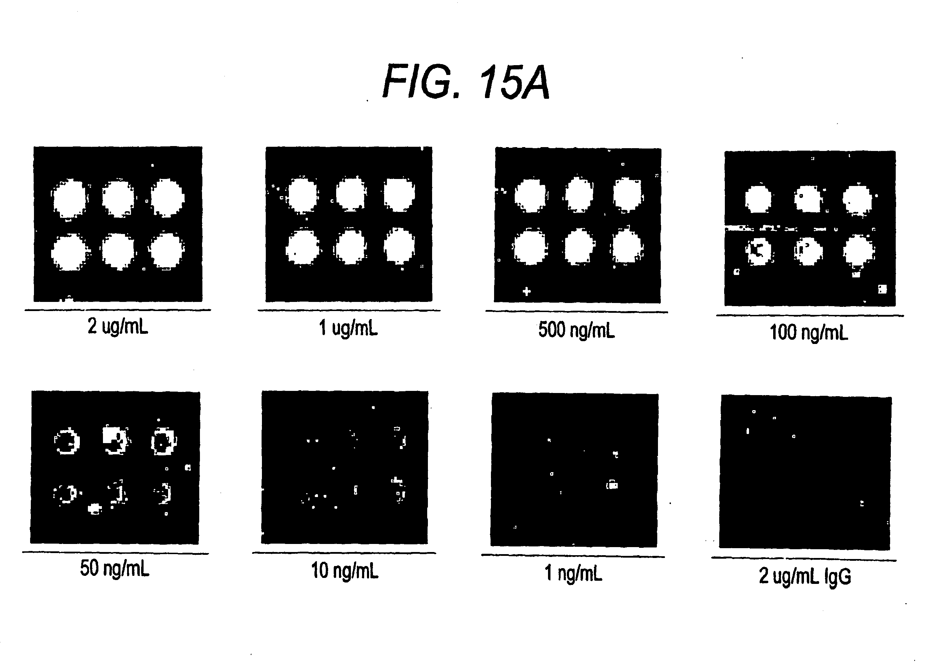

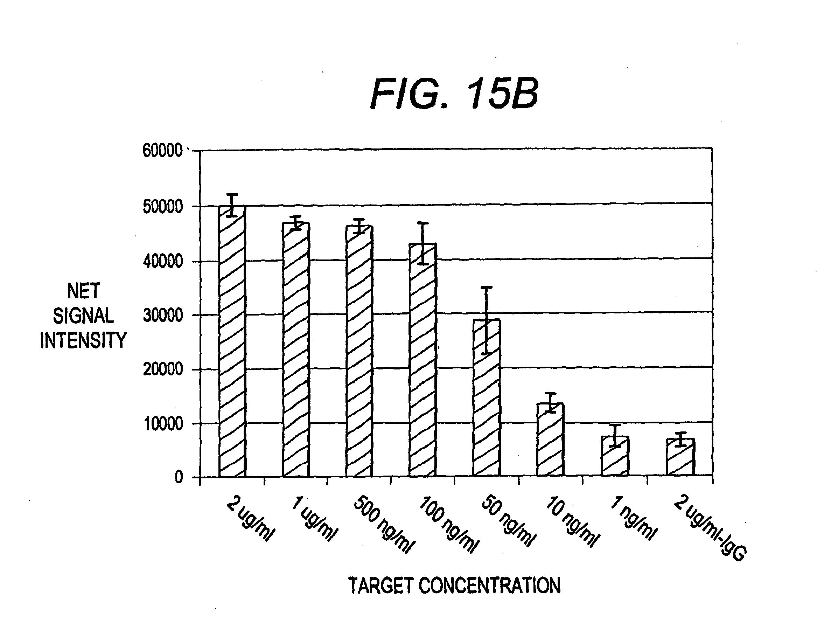

Figure 15 illustrates (a) images of human IgE assays on anti-IgE aptamer coated gold probe arrays recorded using the Verigene ID detection system. The Verigene ID detection system illuminates the slide with a red light emitting diode and captures an image of the slide with a monochrome photosensor. The images were taken after the slide was washed to remove gold probes without target. The human IgE target concentrations are shown below each image. (b) Quantitation of signal from the anti-IgE aptamer coated gold probes on the probe array. Images recorded with the Verigene ID were analyzed using Axon Genepix software. The net signal intensity was calculated by subtracting background signal from the gold probe array spots. The average net signal intensity and standard deviations from the six spots on each array are shown.

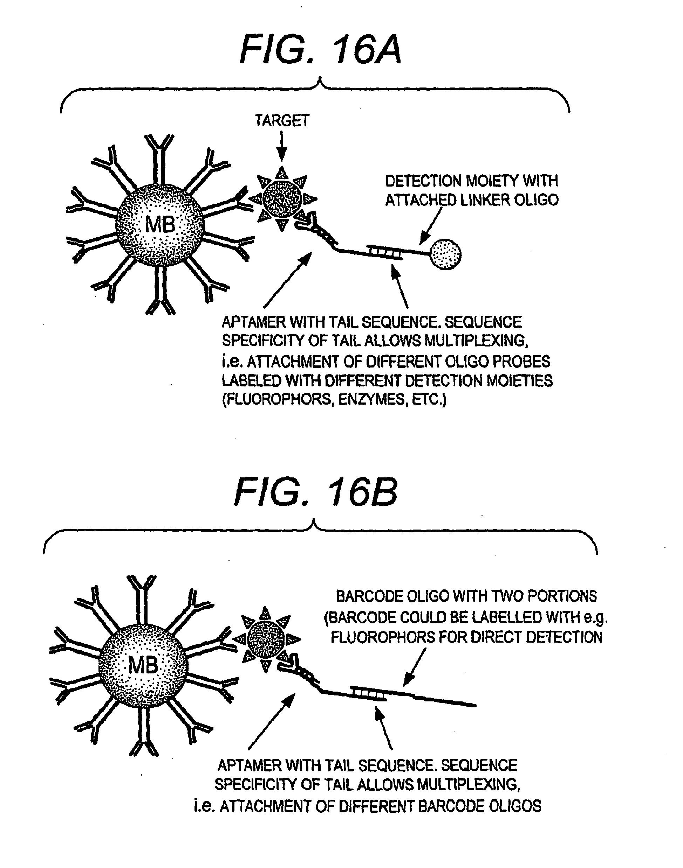

Figure 16 illustrates: (a) the application of an aptamer probe having an oligonucleotide tail hybridized to a linker oligonucleotide labeled with a detection moiety in a sandwich-type assay involving a capture substrate (e.g., a magnetic bead MB), and a target analyte. The design of the aptamer probe allows for multiplex sandwich assays. (b) the oligonucleotide tail of the aptamer is hybridized to at least one portion of linker oligonucleotde or DNA biobarcode. The DNA biobarcode can be optionally labeled with a detectable label, e.g., fluorophore for direct detection or it captured on an array plate and detected using a detection probe.

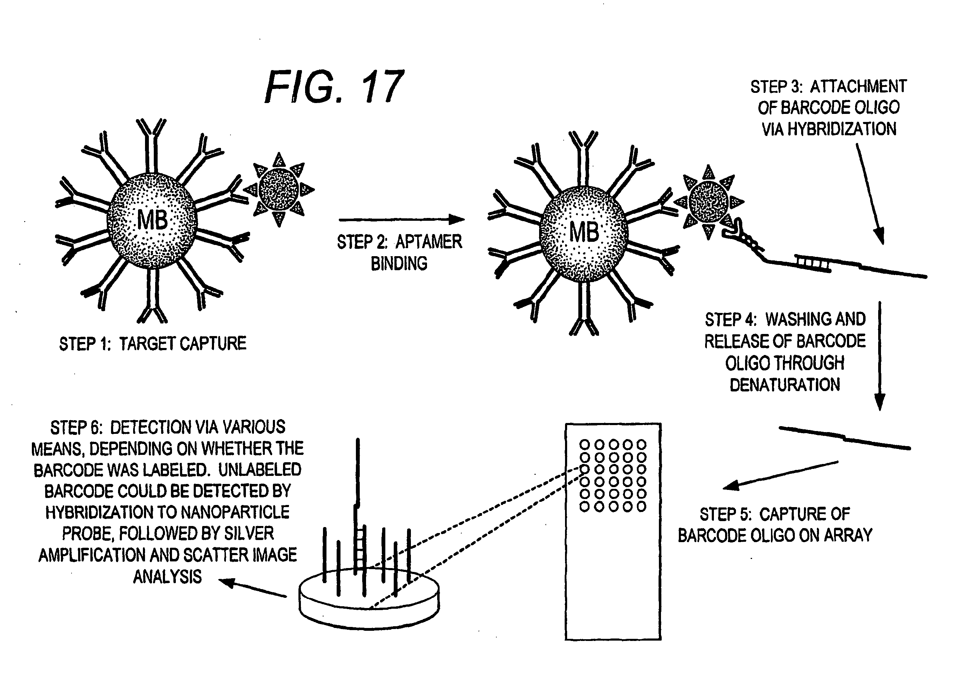

Figure 17 illustrates the capture of a DNA biobarcode on an array plate subsequent to isolation of the sandwiched hybridization complex, denaturation of the complex to release biobarcode. The captured DNA biobarcode can be detected by any suitable means including nanoparticle detection.

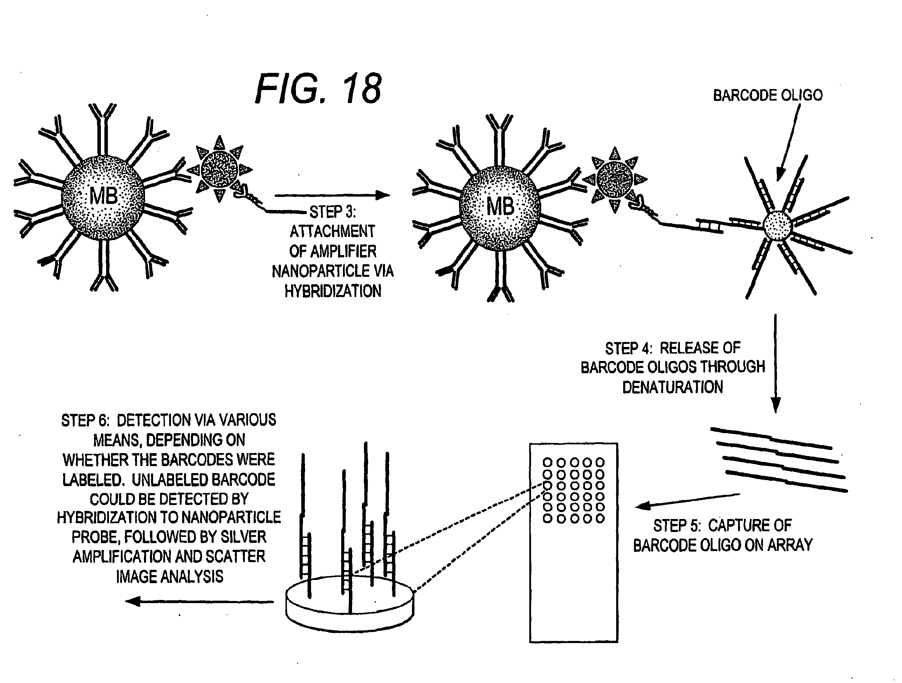

Figure 18 illustrates aptamer-mediated biobarcode-type assay detection employing particle-mediated, e.g., nanoparticle, barcode mediated signal amplification. The released biobarcodes can be detected by any suitable means, depending on whether the barcodes are labeled. Unlabeled barcodes may detected using nanoparticle-labeled detection probes followed by silver amplification.

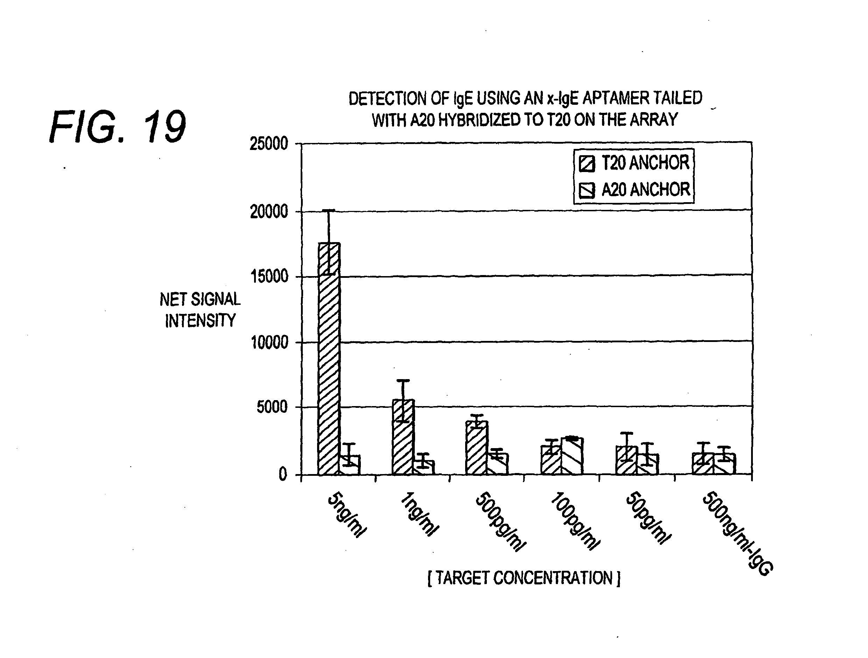

Figure 19. illustrates detection of IgE using an xIgE aptamer tailed with A20 hybridized to T20 to the slide surface.

### DETAILED DESCRIPTION OF THE PREFERRED EMBODIMENTS

Unless otherwise required by context, singular terms shall include pluralities and plural terms shall include the singular.

As utilized in accordance with the present disclosure, the following terms, unless otherwise indicated, shall be understood to have the following meanings:

As used herein, a "nucleic acid sequence," a "nucleic acid molecule," or "nucleic acids" refers to one or more oligonucleotides or polynucleotides as defined herein.

The term "polynucleotide" as referred to herein means single-stranded or doublestranded nucleic acid polymers of at least 10 bases in length. In certain embodiments, the nucleotides comprising the polynucleotide can be ribonucleotides or deoxyribonucleotides or a modified form of either type of nucleotide. Said modifications include base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate. The term "polynucleotide" specifically includes single and double stranded forms of DNA.

The term "oligonucleotide" referred to herein includes naturally occurring, and modified nucleotides linked together by naturally occurring, and/or non-naturally occurring oligonucleotide linkages. Oligonucleotides are a polynucleotide subset comprising members that are generally single-stranded and have a length of 200 bases or fewer. In certain embodiments, oligonucleotides are 10 to 60 bases in length. In certain embodiments, oligonucleotides are 10, 11, 12, 13, 14, 15, 16, 17, 18, 19, or 20 to 40 bases in length. Oligonucleotides may be single stranded or double stranded, *e.g.* for use in the construction of a gene mutant. Oligonucleotides of the invention may be sense or antisense oligonucleotides with reference to a protein-coding sequence.

The term "naturally occurring nucleotides" includes deoxyribonucleotides and ribonucleotides. The term "modified nucleotides" includes nucleotides with modified or substituted sugar groups and the like. The term "oligonucleotide linkages" includes oligonucleotide linkages such as phosphorothioate, phosphorodithioate, phosphoroselenoate, phosphorodiselenoate, phosphoroanilothioate, phoshoraniladate, phosphoroamidate, and the like. *See, e.g.,* LaPlanche et al., 1986, Nucl. Acids Res., 14:9081; Stec et al., 1984, J. Am. Chem. Soc., 106:6077; Stein et al., 1988, Nucl. Acids Res., 16:3209; Zon et al., 1991, Anti-Cancer Drug Design, 6:539; Zon et al., 1991, OLIGONUCLEOTIDES AND ANALOGUES: A PRACTICAL APPROACH, pp. 87-108 (F. Eckstein, Ed.), Oxford University Press, Oxford England; Stec et al., U.S. Pat. No. 5,151,510; Uhlmann and Peyman, 1990, Chemical Reviews, 90:543. An oligonucleotide can include a detectable label to enable detection of the oligonucleotide or hybridization thereof.

An "addressable substrate" used in a method of the invention can be any surface capable of having aptamers or analytes bound thereto. Such surfaces include, but are not limited to, glass, metal, plastic, or materials coated with a functional group designed for binding of aptamers or analytes. The coating may be thicker than a monomolecular layer, in fact, the coating could involve porous materials of sufficient thickness to generate a porous 3-dimensional structure into which the aptamers or analytes can diffuse and bind to the internal surfaces.

The term "capture probe" refers to an aptamer or an antibody. Target analytes such as proteins, polypeptides, fragments, variants, and derivatives may be used to prepare antibodies using methods known in the art. Thus, antibodies and antibody fragments that bind to target analytes may be used in sandwich assays as a capture probe on a substrate when aptamer detection probes are used, as a detection probe when aptamer is used as a capture probe on a substrate, or as detection probes in combination with an aptamer detection probe. Antibodies may be polyclonal, monospecific polyclonal, monoclonal, recombinant, chimeric, humanized, fully human, single chain and/or bispecific.

Polyclonal antibodies directed toward a target analyte generally are raised in animals (e.g., rabbits or mice) by multiple subcutaneous or intraperitoneal injections of JNK activating phosphatase polypeptide and an adjuvant. It may be useful to conjugate an target analyte protein, polypeptide, or a variant, fragment or derivative thereof to a carrier protein that is immunogenic in the species to be immunized, such as keyhole limpet heocyanin, serum, albumin, bovine thyroglobulin, or soybean trypsin inhibitor. Also, aggregating agents such as alum are used to enhance the immune response. After immunization, the animals are bled and the serum is assayed for anti-target analyte antibody titer.

Monoclonal antibodies directed toward target analytes are produced using any method that provides for the production of antibody molecules by continuous cell lines in culture. Examples of suitable methods for preparing monoclonal antibodies include hybridoma methods of Kohler, et al., Nature 256:495-97 (1975), and the human B-cell hybridoma method, Kozbor, J. Immunol. 133:3001 (1984); Brodeur et al., Monoclonal Antibody Production Techniques and Applications 51-63 (Marcel Dekker 1987).

The aptamer containing probes may be employed in any known assay method, such as competitive binding assays, direct and indirect sandwich assays, and immunoprecipitation assays (Sola, Monoclonal Antibodies: A Manual of Techniques 147-58 (CRC Press 1987)) for detection and quantitation of target analytes.

Competitive binding assays rely on the ability of a labeled standard (e.g., an known target polypeptide, or an immunologically reactive portion thereof) to compete with the test sample analyte (an target polypeptide) for binding with a limited amount of anti target antibody. The amount of target analyte in the test sample is inversely proportional to the amount of standard that becomes bound to the antibodies. To facilitate determining the amount of standard that becomes bound, the antibodies typically are insolubilized before or after the competition, so that the standard and analyte that are bound to the antibodies may conveniently be separated from the standard and analyte which remain unbound.

Sandwich assays generally involve the use of two antibodies, each capable of binding to a different immunogenic portion, or epitope, of the protein to be detected and/or quantitated. In a sandwich assay, the test sample analyte is typically bound by a first antibody which is immobilized on a solid support, and thereafter a second antibody binds to the analyte, thus forming an insoluble three part complex. *See, e.g.,* U.S. Patent No. 4,376,110. The second antibody may itself be labeled with a detectable moiety (direct sandwich assays) or may be measured using an anti-immunoglobulin antibody that is labeled with a detectable moiety (indirect sandwich assays). For example, one type of sandwich assay is an ELISA assay, in which case the detectable moiety is an enzyme. In practicing this invention, either the first antibody, the second antibody, or both are replaced with an aptamer.

The term "capture aptamer" as used herein refers to an aptamer that is bound to a substrate and comprises a configuration that can locate (*i.e.* bind in a sample) a target analyte, thereby causing the target non-nucleic acid analyte to be attached to the substrate via the capture aptamer upon binding.

The term "aptamers" refers to nucleic acids (typically DNA, RNA or oligonucleotides) that emerge from in vitro selections or other types of aptamer selection procedures well known in the art (e.g. bead-based selection with flow cytometry or high density aptamer arrays) when the nucleic acid is added to mixtures of molecules. Ligands that bind aptamers include but are not limited to small molecules, peptides, proteins, carbohydrates, hormones, sugar, metabolic byproducts, cofactors, drugs and toxins. Aptamers of the invention are preferably specific for a particular analyte. Aptamers can have diagnostic, target validation and therapeutic applications. The specificity of the binding is defined in terms of the dissociation constant Kd of the aptamer for its ligand. Aptamers can have high affinity with Kd range similar to antibody (pM to nM) and specificity similar/superior to antibody (Tuerk and Gold, 1990, Science, 249:505; Ellington and Szostak, 1990, Nature 346:818). An aptamer will typically be between 10 and 300 nucleotides in length. RNAs and DNAs aptamers can be generated from in vitro selection experiments such as SELEX (Systematic Evolution of Ligands by Exponential Enrichment). Examples of aptamer uses and technology are PhotoSELEX^{™} and Riboreporters^{™}. Aptamers, their uses, and manufacture are described, for example, in U.S. Patent Nos. 5,840,867, 6,001,648, 6225,058, 6,207,388 and U.S. Patent publication 20020001810, the disclosures of all of which are incorporated by reference in their entireties.

Aptamers configured to bind to specific target analytes can be selected, for example, by synthesizing an initial heterogeneous population of oligonucleotides, and then selecting oligonucleotides within the population that bind tightly to a particular target analyte. Once an aptamer that binds to a particular target molecule has been identified, it can be replicated using a variety of techniques known in biological and other arts, for example, by cloning and polymerase chain reaction (PCR) amplification followed by transcription.

The synthesis of a heterogeneous population of oligonucleotides and the selection of aptamers within that population can be accomplished using a procedure known as the Systematic Evolution of Ligands by Exponential Enrichment or SELEX. The SELEX method is described in, for example, Gold et al., U.S. Patent Nos. 5,270,163 and 5,567,588; Fitzwater et al., "A SELEX Primer," Methods in Enzymology, 267:275-301 (1996); and in Ellington and Szostak, "In Vitro Selection of RNA Molecules that Bind Specific Ligands," Nature, 346:818-22. For example, a heterogeneous DNA oligomer population can be synthesized to provide candidate oligomers for the *in vitro* selection of aptamers. The initial DNA oligomer population is a set of random sequences 15 to 100 nucleotides in length flanked by fixed 5' and 3' sequences 10 to 50 nucleotides in length. The fixed regions provide sites for PCR primer hybridization and, in one implementation, for initiation of transcription by an RNA polymerase to produce a population of RNA oligomers. The fixed regions also contain restriction sites for cloning selected aptamers. Many examples of fixed regions can be used in aptamer evolution. See, *e.g.*, Conrad et al., "In Vitro Selection of Nucleic Acid Aptamers That Bind Proteins," Methods in Enzymology, 267:336-83 (1996); Ciesiolka et al., "Affinity Selection-Amplification from Randomized Ribooligonucleotide Pools," Methods in Enzymology, 267:315-35 (1996); and Fitzwater et al., "A SELEX Primer," Methods in Enzymology, 267:275-301 (1996).

Aptamers are selected in a 5 to 100 cycle procedure. In each cycle, oligomers are bound to the target molecule, purified by isolating the target to which they are bound, released from the target, and then replicated by 20 to 30 generations of PCR amplification.

Various oligomers can be used for aptamer selection, including, but not limited to, 2'-fluoro-ribonucleotide oligomers, NH₂-substituted and OCH₃-substituted ribose aptamers, and deoxyribose aptamers. RNA and DNA populations are equally capable of providing aptamers configured to bind to any type of target molecule. Within either population, the selected aptamers occur at a frequency of 10⁹ to 10¹³, see Gold et al., "Diversity of Oligonucleotide Functions," Annual Review of Biochemistry, 64:763-97 (1995), and most frequently have nanomolar binding affinities to the target, affinities as strong as those of antibodies to cognate antigens. See Griffiths et al., EMBO J., 13:3245-60 (1994).

Using 2'-fluoro-ribonucleotide oligomers is likely to increase binding affinities ten to one hundred fold over those obtained with unsubstituted ribo- or deoxyribo-oligonucleotides (see Pagratis et al., "Potent 2'-amino and 2' fluoro 2'deoxyribonucleotide RNA inhibitors of keratinocyte growth factor" Nature Biotechnology, 15:68-73). Such modified bases provide additional binding interactions and increase the stability of aptamer secondary structures. These modifications also make the aptamers resistant to nucleases, a significant advantage for real world applications of the system. See Lin et al., "Modified RNA sequence pools for in vitro selection" Nucleic Acids Research, 22:5229-34 (1994); and Pagratis, et al., "Potent 2'-amino and 2' fluoro 2'deoxyribonucleotide RNA inhibitors of keratinocyte growth factor" Nature Biotechnology, 15:68-73.

The aptamers may include suitable modifications that would allow the aptamer to be attached or bound to a substrate. Suitable, but non-limiting modifications include functional groups such as thiols, amines, carboxylic acids, maleimide, and dienes. Other methods such as hapten interactions may be used. Examples of hapten interactions include, but are not limited to, strepatavidin-biotin, x-biotin-biotin, x-fluorescein/fluorescein and other hapten pairs well known in the art. The aptamers can be prepared by any suitable means, including chemical synthesis and chemical synthesis on solid support.

The aptamers may include an oligonucleotide tail. The term "oligonucleotide tail" refers to a synthetic oligonucleotide extension of the aptamer. This extension may be created during the synthesis of the aptamer or may be added to the 3' or 5' end of the aptamer using any suitable means including chemical or enzymatic means. It is important to note that this extension is added after the aptamer sequence has been selected. Thus, it does not present a part o the sequence that determines the binding activity of the aptamer. The extension is generally single-stranded and has a length of about 10 to 60 bases. In certain embodiments, the oligonucleotide are 10, 11, 12, 13, 14, 15, 16, 17, 18, 19, and 20 to 40 bases in length. The oligonucleotide tail may be any suitable length and sequence that does not interfere with the ability of the aptamer to bind to its target. The oligonucleotide tail has a predetermined sequence, allowing for modification of the aptamer to include any desired label by hybridizing an labeled oligonucleotide, e.g., a fluorophore labeled oligonucleotide, having a sequence that is complementary to at least a portion of the oligonucleotide tail.

An aptamer probe is provided. The aptamer probe comprising: an aptamer having an oligonucleotide tail; and a second linker oligonucleotide having a sequence that is complementary to at least a portion of a sequence of the oligonucleotide tail, said second oligonucleotide having an optional label. The oligonucleotide tail advantageously allows for multiplexing, e.g., the attaching of different oligonucleotide probes labeled with different detection moieties such as fluorophores, dendrimers, radiolabels, enzymes, and the like. The aptamer probe having the oligonucleotide tail is broadly useful in a variety of assays for detecting target analytes, including direct or indirect sandwich assays. Figure 16 illustrates a representative sandwich assay involving a a capture substrate, e.g., a magnetic bead (MB) having capture antibody probes, an detection probe having a detector aptamer having an oligonucleotide tail hybridized to a linker oligonucleotide labeled with a detection moieity, and a target analyte sandwiched between the capture probe and detector aptamer. Figure 16 (b) is the same as Figure 16 (a) but illustrates the use of a linker oligonucleotide as a single DNA biobarcode. The DNA biobarcode can be optionally amplified, e.g., PCR amplification, and detected by any suitable means such as a sandwich assay. Preferably, the DNA biobarcode is detected using a sensitive nanoparticle-based detection system using arrayed substrate and silver amplification (Figure 17). See, U.S. Patent No. 6,750,016 and U.S. serial no. 2005/0037397, filed June 25, 2004. Alternatively, the DNA biobarcode can be labeled with any suitable detection label such a fluorophore. The labeled DNA biobarcode can be detected by any suitable means, including solution-based fluorometry.

In one aspect, the optional label is a detection label that allows detection by photonic, electronic, acoustic, opto-acoustic, gravity, electro-chemical, electro-optic, mass-spectrometric, enzymatic, chemical, biochemical, or physical means. Representative examples include fluorescent, luminescent, phosphorescent, or radioactive detection labels, a quantum dot, a nanoparticle, a dendrimer, a molecular aggregate or a bead, a nanoparticle, and an oligonucleotide having a know sequence. The oligonucleotide is designed to be amplified by physical, chemical or biochemical means such as hybridization cascades or enzymatic means.In another aspect, the optional label is a particle - oligonucleotide conjugate. The particle conjugate label comprises particles having one or more types of DNA barcodes bound directly or indirectly to the nanoparticles. These "DNA barcodes" are oligonucleotides that serve as a surrogate for the target analyte and provide a means for signal amplification. The barcodes that are released may be detected by any suitable means including arrayed substrates in a sandwich assay using any suitable detection probe. The particles may be of any suitable size including nanoparticles and microsized particles, e.g., 1 um, and may be made of any suitable material such as polymers (e.g., polystyrene), metals (e.g., gold or silver), ceramics, semiconductor material.

When the optional label is a particle-oligonucleotide conjugate, the aptamer probe of the invention are particularly useful in biobarcode detection assays such as the one described in U.S. serial number US200505037397, filed June 25, 2004

In the case of protein detection, there are very few methods comparable to PCR that allows one to amplify the signal associated with a protein recognition event. The most promising are immuno-PCR (T. Sano, C.L. Smith, C.R. Cantor, Science 258, 120 (1992)) and the bio-bar-code amplification (Nam, J.M., Thaxton, C.S., Mirkin, C.A. (2003) Science 301, 1884-1886; and Nam, J.M. Stoeva, S.I, Mirkin, C.A. (2004) J. Am. Chem. Soc. 126, 5932-5933) approach to protein detection. The bio-bar-code amplification approach has the advantage that it is higher in sensitivity than immuno-PCR for the systems studied thus far, does not rely on enzymatic amplification and is less complex. For a description of the biobarcode assay, see U.S. serial no. 2005/0037397, filed June 25, 2004 The bio-bar-code amplification assay typically involves two types of particles, a magnetic microparticle (MMP) functionalized with a group that has an affinity for a target of interest and a nanoparticle functionalized with a second group that has an affinity for the same target along with oligonucleotides (bar-code DNA) that can act as reporter groups for the target of interest. When the target is a protein, the recognition agent on the magnetic particle is typically a monoclonal antibody, but may be an aptamer, and the recognition agent on the gold nanoparticle is a polyclonal or monoclonal antibody, but preferably it is an aptamer, that recognizes an epitope distinct from the one on the antibody on the magnetic particle. In the biobarcode assay, the MMP probes are added to a solution containing the protein target of interest. After the MMP probes have been given a chance to react with target, the nanoparticle probes with bar-code DNA are added to form a sandwich structure with the MMP probes that have captured target A suitable separation technique, e.g., a magnetic field, may be used to separate such sandwich complexes from the test solution, and the supernatant is discarded. Dehybridization of the bar-code DNA followed by microarray detection with gold nanoparticle probes allows one to identify the bar-code sequences and quantify the amount of protein target in the test solution. Alternatively, the DNA barcodes bound to the nanoparticles can be further modified with any suitable label (such as a fluorophore) and detected by another suitable means such fluorophore detection methods. Figure 18 illustrates an aptamer-mediated biobarcode assay using the aptamer probe labeled with a nanoparticle-oligonucleotide conjugate having DNA barcodes. The released DNA biocodes can be detected by any suitable means, depending in part on whether the biocodes were labeled or not.

The term "analyte" refers to a substance to be detected or assayed by the method of the invention. Typical analytes may include, but are not limited to proteins, peptides, nucleic acid segments, molecules, cells, microorganisms and fragments and products thereof, or any substance for which attachment sites, binding members or receptors (such as antibodies) can be developed. The analytes have at least one binding site, preferably at least two binding sites, e.g., epitopes, that can be targeted by a capture probe and a detection probe, e.g. antibodies or aptamers or both..

A "detection probe" can be any carrier to which one or more detection aptamers or antibodies can be attached, wherein the one or more detection aptamers comprise a configuration that binds a specific target analyte. The carrier itself may serve as a label, or may contain or be modified with a detectable label, or the detection aptamers may carry such labels. Carriers that are suitable for the methods of the invention include, but are not limited to, nanoparticles, quantum dots, dendrimers, semiconductors, beads, up- or down-converting phosphors, large proteins, lipids, carbohydrates, or any suitable inorganic or organic molecule of sufficient size, or a combination thereof.

A carrier a nanoparticle. Nanoparticles useful include metal (e.g., gold, silver, copper and platinum), semiconductor (e.g., CdSe, CdS, and CdS or CdSe coated with ZnS) and magnetic (e.g., ferromagnetite) colloidal materials. Other useful nanoparticles include Zns, ZnO, TiO₂, AgI, AgBr, HgI_{2,} PbS, PbSe, ZnTe, CdTe, In₂ S₃, In₂ Se₃, Cd₃ P₂, Cd₃ As₂, InAs, and GaAs. The size of the nanoparticles is preferably from about 5 nm to about 150 nm (mean diameter), more preferably from about 5 to about 50 nm, most preferably from about 10 to about 30 nm. The nanoparticles may also be rods. Other nanoparticles useful in the invention include silica and polymer (e.g. latex) nanoparticles.

Methods of making metal, semiconductor and magnetic nanoparticles are well-known in the art. See, e.g., Schmid, G. (ed.) Clusters and Colloids (VCH, Weinheim, 1994); Hayat, M. A. (ed.) Colloidal Gold: Principles, Methods, and Applications (Academic Press, San Diego, 1991); Massart, R., IEEE Taransactions On Magnetics, 17, 1247 (1981); Ahmadi, T. S. et al., Science, 272, 1924 (1996); Henglein, A. et al., J. Phys. Chem., 99, 14129 (1995); Curtis, A. C., et al., Angew. Chem. Int. Ed. Engl., 27, 1530 (1988). Methods of making silica nanoparticles impregnated with fluorophores or phosphors are also well known in the art (see Tan and coworkers, PNAS, 2004, 101, 15027-15032).

Methods of making ZnS, ZnO, TiO₂, AgI, AgBr, HgI₂, PbS, PbSe, ZnTe, CdTe, In₂ S₃, In₂ Se₃, Cd₃ P₂, Cd₃ As₂, InAs, and GaAs nanoparticles are also known in the art. See, e.g., Weller, Angew. Chem. Int. Ed. Engl., 32, 41 (1993); Henglein, Top. Curr. Chem., 143, 113 (1988); Henglein, Chem. Rev., 89, 1861 (1989); Brus, Appl. Phys. A., 53, 465 (1991); Bahncmann, in Photochemical Conversion and Storage of Solar Energy (eds. Pelizetti and Schiavello 1991), page 251; Wang and Herron, J. Phys. Chem., 95, 525 (1991); Olshavsky et al., J. Am. Chem. Soc., 112, 9438 (1990); Ushida et al., J. Phys. Chem., 95, 5382 (1992).

Suitable nanoparticles are also commercially available from, e.g., Ted Pella, Inc. (gold), Amersham Corporation (gold), Nanoprobes, Inc. (gold), and Quantom Dot Inc. (core-shell semiconductor particles such as CdSe/ZnS).

A nanoparticle can have a zero, one, or a plurality of diluent oligonucleotides, as well as aptamers, attached to it. For example, nanoparticles can be incubated with aptamers and oligonucleotides in a 3:1 ratio, as described in the Examples below. In one embodiment, the oligonucleotides are polyadenosine oligonucleotides, for example A₁₀, which is an oligonucleotide consisting of 10 adenosines. In another embodiment, the oligonucleotide consists of 20 adenosines. The use of diluent oligonucleotides in addition to aptamers provides a means of tailoring the conjugates to give a desired level of binding interaction. The diluent and aptamers have been found to attach to the nanoparticles in about the same proportion as their ratio in the solution contacted with the nanoparticles to prepare the conjugates. Thus, the ratio of the diluent to aptamers bound to the nanoparticles can be controlled so that the conjugates will participate in a desired number of binding events. The diluent oligonucleotides may have any sequence which does not interfere with the ability of the aptamer to be bound to the nanoparticles or to bind to a target analyte. For instance, the diluent oligonulceotides should not have a sequence complementary to that of the aptamer or a nucleic acid target analyte. The diluent oligonucleotides are also preferably of a length shorter than that of the aptamer so that the aptamers can bind to their targets without interfering with the ability of the aptamers to bind with their respective targets.

As used herein, a "detector aptamer" or "detection aptamer," is an aptamer as defined herein that comprises a configuration that can be used to locate (*i.e.* bind in a sample) a target analyte.

As used herein, the terms "label" or "detection label" refers to a detectable marker that may be detected by photonic, electronic, opto-electronic, magnetic, gravity, acoustic, enzymatic, or other physical or chemical means. The term "labeled" refers to incorporation of such a detectable marker, *e.g*., by incorporation of a radiolabeled nucleotide or attachment to an aptamer of a detectable marker.

In another embodiment, a detector oligonucleotide can be detectably labeled. Various methods of labeling polynucleotides are known in the art and may be used advantageously in the methods disclosed herein. In a particular embodiment, a detectable label of the invention can be fluorescent, luminescent, Raman active, phosphorescent, radioactive, or efficient in scattering light, have a unique mass, or other has some other easily and specifically detectable physical or chemical property, and in order to enhance said detectable property the label can be aggregated or can be attached in one or more copies to a carrier, such as a dendrimer, a molecular aggregate, a quantum dot, or a bead. The label can allow for detection, for example, by photonic, electronic, acoustic, opto-acoustic, gravity, electro-chemical, enzymatic, chemical, Raman, or mass-spectrometric means.

A "sample" as used herein refers to any quantity of a substance that comprises nucleic acids and that can be used in a method of the invention. For example, the sample can be a biological sample or can be extracted from a biological sample derived from humans, animals, plants, fungi, yeast, bacteria, viruses, tissue cultures or viral cultures, or a combination of the above. They may contain or be extracted from solid tissues (e.g. bone marrow, lymph nodes, brain, skin), body fluids (e.g. serum, blood, urine, sputum, seminal or lymph fluids), skeletal tissues, or individual cells. Alternatively, the sample can comprise purified or partially purified nucleic acid molecules and, for example, buffers and/or reagents that are used to generate appropriate conditions for successfully performing a method of the invention.

A detector probe of the invention can be a nanoparticle probe having at least one type of detector aptamers bound thereto. Two or more types of aptamers may also be used for multiplex assays involving more than one target analyte. Any suitable surface density of detector aptamer may be used, however, a surface density ranging from between about 8.9 x 10¹¹ and about 6.4 x 10¹² aptamers/cm² was found to be useful. If desired, the nanoparticle probe having detector aptamers bound thereto may further comprise oligonucleotides such as poly adenosine A10 or A20 oligonucleotides. Thioaptamers having phosphorothioate or phosphorodithioate functional moieties are preferred. Any suitable nanoparticle label such as metallic nanoparticles or semiconductor nanoparticles may be used. A particularly preferred nanoparticle of noble metal, e.g., gold, may be used.

Nanoparticles have been a subject of intense interest owing to their unique physical and chemical properties that stem from their size. Due to these properties, nanoparticles offer a promising pathway for the development of new types of biological sensors that are more sensitive, more specific, and more cost effective than conventional detection methods. Methods for synthesizing nanoparticles and methodologies for studying their resulting properties have been widely developed over the past 10 years (Klabunde, editor, Nanoscale Materials in Chemistry, Wiley Interscience, 2001). However, their use in biological sensing has been limited by the lack of robust methods for functionalizing nanoparticles with biological molecules of interest due to the inherent incompatibilities of these two disparate materials. A highly effective method for functionalizing nanoparticles with modified oligonucleotides has been developed. See U.S. Patent Nos. 6,361,944 and 6,417,340 (assignee: Nanosphere, Inc.).

The process leads to nanoparticles that are heavily functionalized with oligonucleotides, which have surprising particle stability and hybridization properties. The resulting DNA-modified particles have also proven to be very robust as evidenced by their stability in solutions containing elevated electrolyte concentrations, stability towards centrifugation or freezing, and thermal stability when repeatedly heated and cooled. This loading process also is controllable and adaptable. Such methods can also be used to generate nanoparticle-aptamer conjugates.

Nanoparticles of differing size and composition have been functionalized, and the loading of oligonucleotide recognition sequences onto the nanoparticle can be controlled via the loading process. Suitable, but non-limiting examples of nanoparticles include those described U.S. Patent No. 6,506,564; International Patent Application No. WO 2003/008539 ; U.S. Patent Application Serial No. 2004/0086897 filed May 7, 2003; and International Patent Application No. WO 2003/095973.

Nanoparticles having bound thereto aptamers and optional diluent oligonucleotides are preferably prepared by a salt aging method for preparing nanoparticle-oligonucleotide conjugates as described in U.S. Patent no. 6,506,564, which is incorporated by reference in its entirety. Aptamers and oligonucleotides having covalently bound thereto a moiety comprising a functional group which can bind to the nanoparticles are used. The moieties and functional groups are those described in U.S. Patent nos. 6,506,564 and 6,767,702 for binding (*i.e*., by chemisorption or covalent bonding) oligonucleotides to nanoparticles. For instance, oligonucleotides having an alkanethiol or an alkanedisulfide covalently bound to their 5' or 3' ends can be used to bind the oligonucleotides to a variety of nanoparticles, including gold nanoparticles. Thioaptameis having phosphorothioate or phosphorodithioate functional moieties covalently bound to their 5' or 3' ends can be used to bind the aptamers to a variety of nanoparticles, including gold nanoparticles. Additionally, the oligonucleotides can be bound through an oligonucleotide tail such as a polyA tail which has a high affinity for the gold nanoparticle surface (see Tarlov and coworkers, JACS, 2004). Alternatively, streptavidin or x-biotin modified nanoparticles can be contacted with biotinylated aptamers to form the aptamer nanoparticle conjugate.

The aptamers and optional diluent oligonucleotides are contacted with the nanoparticles in water for a time sufficient to allow at least some of the aptamers and oligonucleotides to bind to the nanoparticles by means of the functional groups. Such times can be determined empirically. For instance, it has been found that a time of about 12-24 hours gives good results. Other suitable conditions for binding of the aptamers and oligonucleotides can also be determined empirically. For instance, a concentration of about 10-20 nM nanoparticles and incubation at room temperature gives good results.

Next, at least one salt is added to the water to form a salt solution. The salt can be any water-soluble salt. For instance, the salt may be sodium chloride, magnesium chloride, potassium chloride, ammonium chloride, sodium acetate, ammonium acetate, a combination of two or more of these salts, or one of these salts in phosphate buffer. Preferably, the salt is added as a concentrated solution, but it could be added as a solid. The salt can be added to the water all at one time or the salt is added gradually over time. By "gradually over time" is meant that the salt is added in at least two portions at intervals spaced apart by a period of time. Suitable time intervals can be determined empirically.

The ionic strength of the salt solution must be sufficient to overcome at least partially the electrostatic repulsion of the oligonucleotides from each other and, either the electrostatic attraction of the negatively-charged oligonucleotides for positively-charged nanoparticles, or the electrostatic repulsion of the negatively-charged oligonucleotides from negatively-charged nanoparticles. Gradually reducing the electrostatic attraction and repulsion by adding the salt gradually over time has been found to give the highest surface density of oligonucleotides on the nanoparticles. Suitable ionic strengths can be determined empirically for each salt or combination of salts. A final concentration of sodium chloride of from about 0.1 M to about 1.0 M in phosphate buffer, preferably with the concentration of sodium chloride being increased gradually over time, has been found to give good results.

After adding the salt, the aptamers, oligonucleotides and nanoparticles are incubated in the salt solution for an additional period of time sufficient to allow sufficient additional oligonucleotides to bind to the nanoparticles to produce the stable nanoparticle conjugates having aptamers and oligonucleotides bound thereto. As will be described in detail below, an increased surface density of the oligonucleotides on the nanoparticles has been found to stabilize the conjugates. The time of this incubation can be determined empirically. A total incubation time of about 24-48, preferably 40 hours, has been found to give good results (this is the total time of incubation; as noted above, the salt concentration can be increased gradually over this total time). This second period of incubation in the salt solution is referred to herein as the "aging" step. Other suitable conditions for this "aging" step can also be determined empirically. For instance, incubation at room temperature and pH 7.0 gives good results.

The aptamer nanoparticle conjugates produced by use of the "aging" step have been found to be considerably more stable than those produced without the "aging" step. As noted above, this increased stability is due to the increased density of the oligonucleotides on the surfaces of the nanoparticles which is achieved by the "aging" step. The surface density achieved by the "aging" step will depend on the size and type of nanoparticles and on the length, sequence and concentration of the aptamers/oligonucleotides. A surface density adequate to make the nanoparticles stable and the conditions necessary to obtain it for a desired combination of nanoparticles and aptamers/oligonucleotides can be determined empirically.

The aforementioned loading method for preparing DNA-modified nanoparticles, particularly aptamer-modified gold nanoparticle probes, has led to the development of a colorimetric sensing scheme for oligonucleotides and non-nucleic acid targets. See See, for instance, U.S. Patent No. 6,506,5641 describes a colorimetric sensing scheme based on DNA-modified nanoparticles. This method is based on the hybridization of two gold nanoparticle probes to two distinct regions of a target, e.g., DNA, of interest. Since each of the probes are functionalized with multiple oligonucleotides bearing the same sequence, the binding of the target results in the formation of target/gold nanoparticle probe aggregate when sufficient target is present. The DNA target recognition results in a colorimetric transition due to the decrease in interparticle distance of the particles. This colorimetric change can be monitored optically, with a UV-vis spectrophotometer, or visually with the naked eye. In addition, the color is intensified when the solutions are concentrated onto a membrane. Therefore, a simple colorimetric transition provides evidence for the presence or absence of a specific DNA sequence. Using this assay, femtomole quantities and nanomolar concentrations of model DNA targets and polymerase chain reaction (PCR) amplified nucleic acid sequences have been detected.

The development of DNA-modified nanoparticle conjugates, particularly aptamer-modified gold nanoparticle probes, has also led to a colorimetric method for monitoring scattered light for oligonucleotides and non nucleic acid targets. See U.S. Ser. No. 2005/0250094, filed November 22, 2004. The scatter-based colorimetric detection method provides much higher sensitivity (> 4 orders of magnitude) in nucleic acid detection than the previously reported absorbance-based spot test when coupled to an improved hybridization method based on neutral or anionic polysaccharides that enables probe-target binding at low target concentrations. Moreover, the methods of the invention enable the detection of probe-target complexes containing two or more particles in the presence of a significant excess of non-oomplexed particles, which drives hybridization in the presence of low target concentrations. Also, dextran sulfate mediated probe-target complex formation in conjunction with evanescent induced scatter as provided herein enables a simple homogeneous hybridization and colorimetric detection protocol for nucleic acid sequences in total bacterial DNA, or with antibody-antigen interactions.

As described herein, nanoparticle probes, particularly gold nanoparticle probes comprising aptamers, are surprising and unexpectedly suited for detection of analytes. A silver-based signal amplification procedure in a microarray-based assay can further provide ultra-high sensitivity enhancement Silver staining can be employed with any type of nanoparticles that catalyze the reduction of silver. Preferred are nanoparticles made of noble metals (*e.g.,* gold and silver). See Bassell, et al., J. Cell Biol., 126, 863-876 (1994); Braun-Howland et al., Biotechniques, 13, 928-931 (1992). Silver straining can be used to produce or enhance a detectable change in any assay performed on a substrate, including those described above. In particular, silver staining has been found to provide a huge increase in sensitivity for assays employing a single type of nanoparticle so that the use of layers of nanoparticles, aggregate probes and core probes can often be eliminated.

A nanoparticle can be detected in a method of the invention, for example, using an optical or flatbed scanner. The scanner can be linked to a computer loaded with software capable of calculating grayscale measurements, and the grayscale measurements are calculated to provide a quantitative measure of the amount of analyte detected.

Suitable scanners include those used to scan documents into a computer which are capable of operating in the reflective mode (*e.g*., a flatbed scanner), other devices capable of performing this function or which utilize the same type of optics, any type of greyscale-sensitive measurement device, and standard scanners which have been modified to scan substrates according to the invention.

The software can also provide a color number for colored spots and can generate images (*e.g*., printouts) of the scans, which can be reviewed to provide a qualitative determination of the presence of a nucleic acid, the quantity of a nucleic acid, or both. In addition, it has been found that the sensitivity of assays can be increased by subtracting the color that represents a negative result from the color that represents a positive result.

The computer can be a standard personal computer, which is readily available commercially. Thus, the use of a standard scanner linked to a standard computer loaded with standard software can provide a convenient, easy, inexpensive means of detecting and quantitating nucleic acids when the assays are performed on substrates. The scans can also be stored in the computer to maintain a record of the results for further reference or use. Of course, more sophisticated instruments and software can be used, if desired.

A nanoparticle can be detected in a method, for example, using resonance light scattering, after illumination by various methods including dark-field microscopy, evanescent waveguides, or planar illumination of glass substrates. Metal particles > 40 nm diameter scatter light of a specific color at the surface plasmon resonance frequency (Yguerabide, J.; Yguerabide, E. E. Anal. Biochem. (1998), 262, 157-176) and can be used for multicolor labeling on substrates by controlling particle size, shape, and chemical composition (Taton, T. A.; Lu, G.; Mirkin, C. A. J. Am. Chem. Soc. (2001), 123, 5164-5165; Jin, R. C.; Cao, Y. W.; Mirkin, C. A.; Kelly, K. L.; Schatz, G. C.; Zheng, J. G. Science (2001), 294, 1901-1903). A nanoparticle can be detected in a method, for example, using surface enhanced raman spectroscopy (SERS) in either a homogeneous solution based on nanoparticle aggregation (Graham and coworkers, Angew. Chem., 2000, 112, 1103.) or on substrates in a solid-phase assay (Porter and coworkers, Anal. Chem. ,1999, 71, 4903-4908), or using silver development followed by SERS (Mirkin and coworkers, Science, 2002, 297, 1536-1540).

The nanoparticles detected by photothermal imaging (Boyer et. al, Science, 2002, 297, 1160-1163). The nanoparticles detected by diffraction-based sensing technology (Bailey et. al, J. Am Chem. Soc., 2003, 125, 13541).

The nanoparticles detected by hyper-Rayleigh scattering (Kim et. al, Chem Phys. Lett., 2002, 352, 421).

A aptamers attached to a substrate can be located between two electrodes, the nanoparticles can be made of a material that is a conductor of electricity, and step (d) in the methods can comprise detecting a change in conductivity. A plurality of aptamers, each of which can recognize a different target analyte, are attached to a substrate in an array of spots and each spot of aptamers is located between two electrodes, the nanoparticles are made of a material that is a conductor of electricity, and step (d) in the methods comprises detecting a change in conductivity. The electrodes can be made, for example, of gold and the nanoparticles are made of gold. Alternatively, a substrate can be contacted with silver stain to produce a change in conductivity.

The binding conditions effective for the specific and selective binding of aptamers to a target analyte. A typical single-stranded or doublestranded nucleic acid aptamer has secondary structure that enables a specific binding interaction with the target analyte. Therefore, the conditions used for specific and selective binding of aptamers to a specific target analyte require the aptamer to be folded in a specific conformation. For example, the IgE aptamer used in the provided examples folds into a stem-loop structure under the appropriate pH and salt conditions (e.g. MgCl₂), which facilitates binding of the IgE target. Therefore, the structure of the aptamer and the conditions which produce this structure are an important factor for choosing appropriate assay conditions.

Methods of detecting analytes including non-nucleic acid and nucleic acid molecules. The method comprise contacting an analyte with nanoparticles having aptamers attached thereto (nanoparticle-aptamer conjugates), wherein the aptamers have a configuration capable of binding to specific target analytes.

A method for detecting at least one target analyte in a sample, the target analyte having at least two binding sites, is provided. The method comprises the steps of providing a substrate having at least one type of capture probe bound thereto, wherein the capture probe can bind to a first binding site of a specific target analyte and providing at least one type of nanoparticle probe comprising detector aptamers, wherein the detector aptamers can bind to a second binding site of the target analyte. The method comprisings contacting the sample with the substrate and the nanoparticle probe under conditions that are effective for the binding of the capture probe to the first binding site of the target analyte and the binding of the nanoparticle probe to the second binding site of the target analyte to form a complex. Finally, the presence or absence of the complex may be detected wherein the presence or absence of the complex is indicative of the presence or absence of the specific target analyte.

A method for detecting at least one type of target analyte in a sample, the target analyte having at least two binding sites, is provided. The method comprising the steps of providing at least one type of nanoparticle probe comprising detector aptamers, wherein the detector aptamers on each type of probe has a configuration that can bind to a first binding site of a specific type of target analyte. The sample is contacted with the nanoparticle probes under conditions that are effective for the binding of the detector aptamers to the target analyte. Finally, the determination of whether the detector aptamer binds to the target analyte is made.

A method is provided for detecting a target analyte in a sample, said target analyte having at least two binding sites. The method comprises the steps of providing a type of nanoparticles having aptamers bound thereto, the aptamers capable of binding to two or more binding sites of the target analyte. The sample and the nanoparticles having aptamers bound thereto are contacted under conditions effective to allow binding between the target analyte and the aptamers bound to nanoparticles bound thereto. An observation is then made for a detectable change brought about by the binding of the target analyte with the aptamers bound to the nanoparticles.

A method is provided for detecting a target analyte in a sample, said target analyte having at least two binding sites. The method includes providing at least two types of nanoparticles having aptamers bound thereto, each type of aptamer capable of binding to a different binding site of the target analyte. The sample and the at least two types of nanoparticles having aptamers bound thereto are contacted under conditions effective to allow binding between the target analyte and the aptamers bound to the nanoparticles. Thereafter, an observation is made for a detectable change brought about by the binding of the the aptamers bound to the nanoparticles with the target analyte. The method for detection of specific binding analytes is based on analyte mediated formation of metallic nanoparticle-labeled probe complexes, e.g., gold nanoparticle probe complexes, that results in a change in the color and/or intensity of light scattered, which can be measured by placing a small amount of the sample onto a waveguide and detecting the light scattered visually or with a photosensor. Examples of this detection method applied to nucleic acid detection is discussed in Example 6 below. See also co-pending U.S. serial no. 2005/0250094, filed November 22, 2004. The nanoparticle probe complexes comprise two or more probes bound to a specific target analyte.

A method is provided for detecting a target analyte in a sample, said target analyte having at least two binding sites. The method comprising the steps of providing at least one type of nanoparticles having aptamers bound thereto, the aptamer capable of binding to a binding site of the target analyte and at least one type of nanoparticles having antibodies bound thereto, the antibodies capable to binding to a different binding site of the target analyte. The sample, the nanoparticles having aptamers, and nanoparticles having antibodies bound thereto are contacted under conditions effective to allowi binding between the target analyte and the aptamers and the antibodies bound to the nanoparticles. Thereafter, an observation is made for a detectable change brought about by the binding of the the aptamers bound to the nanoparticles and the antibodies bound to the nanoparticles with the target analyte.

A substrate for detection of one or more target analytes is provided. The substrate includes a substrate, at least one type of capture aptamers bound to the substrate, each type of capture aptamers binds to a specific target analyte and arranged in an array of discrete spots; and (c) optional electrodes located between the discrete spots.

A method is provided for detecting at least one target analyte in a sample, the target analyte having at least two binding sites, the method comprising the steps of: (a) providing a substrate having at least one type of capture probe bound thereto, wherein the capture probe can bind to a first binding site of a specific target analyte; (b) providing at least one type of detector aptamer probe, wherein the detector aptamers can bind to a second binding site of the target analyte; (c) contacting the sample with the substrate and the probe under conditions that are effective for the binding of the capture probe to the first binding site of the target analyte and the binding of the aptamer probe to the second binding site of the target analyte to form a complex; and (d) observing for a detectable change. The captured target-aptamer probe complex is detected by photonic, electronic, acoustic, opto-acoustic, gravity, electro-chemical, electro-optic, mass-spectrometric, enzymatic, chemical, biochemical, or physical means.

In one aspect, the capture probe comprises an antibody or an capture aptamer. The binding sites are epitopes that a specific capture probe binds to.

In another aspect, two or more types of aptamer probes are provided, each type of probes having detector aptamers bound thereto that are capable of binding to a different epitope on the same target analyte or to different target analytes, or both.

In another aspect, sample is first contacted with the aptamer probe so that a target analyte present in the sample binds to the detector aptamers on the probe, and the target analyte bound to the aptamer probe is then contacted with the substrate so that the target ' analyte binds to the capture probe on the substrate.

In another aspect, the sample is first contacted with the substrate so that a target analyte present in the sample binds to a capture probe, and the target analytes bound to the capture aptamer is then contacted with the aptamer probe so that the target analyte binds to the detector aptamers on the aptamer probe.

In another aspect, the sample, the aptamer probe and the capture probe on the substrate are contacted simultaneously.

Any suitable substrate may be used and such substrates may be addressable. Representative substrates are described above. A plurality of capture probes, each of which can recognize a different target analyte, may be attached to the substrate in an array of spots. If desired, each spot of capture probes may located between two electrodes, the optional label on the aptamer probe is a nanoparticle made of a material that is a conductor of electricity, and a change in conductivity may be detected. The electrodes are made of gold and the nanoparticles are made of gold.

A method for detecting at least one type of target analyte in a sample, the target analyte having at least two binding sites, the method comprising the steps of: (a) providing at least one type of detector aptamer probe, wherein the detector aptamers on each type of probe has a configuration that can bind to a first binding site of a specific type of target analyte; (c) contacting the sample with the aptamer probe under conditions that are effective for the binding of the detector aptamers to the target analyte; and (d) detecting whether the detector aptamer binds to the target analyte.

A method is provided for detecting a target analyte in a sample, said target analyte having at least two binding sites, the method comprising the steps of: (a) providing a type of detector aptamer probes of claim 33, the aptamers capable of binding to two or more binding sites of the target analyte; (b) contacting the sample, and the aptamer probes having aptamers bound thereto under conditions effective to allow binding between the target analyte and the aptamers; and (c) observing a detectable change brought about by the binding of the target analyte to the aptamers.

A method for detecting a target analyte in a sample is provided, said target analyte having at least two binding sites, the method comprising the steps of: (a) providing at least two types of aptamer probes of claim 33, each type of aptamer capable of binding to a different binding site of the target analyte; (b) contacting the sample, and the at least two types of aptamers probes under conditions effective to allow binding between the target analyte and the aptamers bound to the nanoparticles; and (c) observing a detectable change brought about by the binding of the the aptamers to the target analyte.

A kit for detecting for one or more analytes in a sample, the kit comprising an aptamer detection probe and an optional substrate. The substrate may be arrayed with at least one capture probe for a specific target analyte.

### EXAMPLES

The invention is demonstrated further by the following illustrative examples. In these examples all percentages are by weight if for solids and by volume if for liquids, and all temperatures are in degrees Celsius unless otherwise noted.

The representative Examples below demonstrate the efficacy and utility of the inventive method for detecting protein analytes based on DNA aptamer modified gold nanoparticles. Previous studies have demonstrated that DNA can be conjugated to gold nanoparticles via a thiol linkage (Mirkin, C. A.; Letsinger, R L.; Mucic, R. C.; Storhoff, J. J. Nature (1996), 382, 607-609). The resulting DNA modified gold particles have been used to detect DNA targets as well as other analytes in a variety of formats (see, for instance, Storhoff, J. J.; Mirkin, C. A. Chem. Rev. (1999), 99, 1849-1862; Niemeyer, C. M. Angew. Chem. Int. Ed (2001), 40, 4128-4158; Liu, J.; Lu, Y. J. Am. Chem. Soc. (2003), 125, 6642-6643), including DNA microarrays, where high detection sensitivity is achieved in conjunction with silver amplification (Taton, T. A.; Mirkin, C. A.; Letsinger, R. L. Science (2000), 289, 1757-1760; Storhoff, J. J.; Marla, S. M.; Bao, P.; Hagenow, S.; Mehta, H.; Lucas, A.; Garimella, V.; Patno, T.; Buckingham, W.; Cork, W.; Muller, U. Biosens. Bioelectron. (2004), 19, 875-883). Additional key features of this technology include the remarkable stability and robustness of the DNA-modified gold nanoparticles which withstand both elevated temperatures and salt concentrations (Mirkin, C. A.; Letsinger, R. L.; Mucic, R. C.; Storhoff, J. J. Nature (1996), 382, 607-609; Storhoff, J. J.; Elghanian, R.; Mirkin, C. A.; Letsinger, R. L. Langmuir (2002), 18, 6666-6670), as well as the remarkable specificity by which DNA sequences are recognized (Storhoff, J. J.; Elghanian, R.; Mucic, R. C.; Mirkin, C. A.; Letsinger, R. L. J. Am. Chem. Soc. (1998), 120, 1959-1964; Taton, T. A.; Mucic, R. C.; Mirkin, C. A.; Letsinger, R. L. J. Am. chem. Soc. (2000), 122, 6305-6306). Although prior studies have demonstrated that antibodies or haptens can be attached to gold nanoparticles through DNA-directed immobilization or passive adsorption and used for protein detection (Nielsen, U. B.; Geierstanger, B. H. Journal Immunol. Meth. (2004), 290, 107-120; Niemeyer, C. M.; Ceyhan, B. Angew. Chem. Int. Ed. (2001), 40, 3585-3688.; Nam, J. M.; Park, S. J.; Mirkin, C. A. J. Am. Chem. Soc. (2002), 124, 3820-3821), these strategies are still prone to the limitations discussed above. It would be a significant advance to use the DNA-modified gold particles directly for protein analyte detection. The Examples also demonstrate that nucleic acid-based aptamers, which have been developed against a variety of protein analytes for both diagnostic and therapeutic applications(Jayasena, S. D. Clin. Chem. (1999), 45, 1628-1650; Brody, E.; Gold, L. Rev. Mol. Biotech.(2000), 74, 5-13; Potyrailo, R. A.; Conrad, R. C.; Ellington, A. D.; Hieftje, G. M. Anal. Chem. (1998), 70, 3419-3425), can be conjugated to gold nanoparticles. In addition, the Examples demonstrate that the resulting aptamer coated gold probes (AGPs) may detect antibody targets with higher specificity and sensitivity than antibody labeled gold probes.

### Example 1. Methods for the preparation of oligonucleotide aptamer derivatized gold nanoparticles and the detection of human IgE (Method no. 1)

In this Example, a representative gold nanoparticle-aptamer oligonucleotide conjugate detection probe was prepared for the use in the detection of IgE protein. Tasset and coworkers originally reported an aptamer oligonucleotide sequence that binds to human IgE with high affinity and high specificity (Wiegand et. al, 1996, The Journal of Immunology, Vol. 157,221-230). Subsequently, an aptamer sequence with an extended stem-loop structure was designed to increase the IgE binding affinity (Liss et. al, 2002, Anal. Chem, Vol. 74, 4488 - 4495). The aptamer sequence and estimated secondary structure from the reported study are outlined in Figure 1. The aptamer sequence shown in Figure 1 was conjugated to gold nanoparticles for use as detection probes using procedures described in PCT/US97/12783, filed July 21, 1997; PCT/US00/17507, filed June 26, 2000; PCT/US01/01190, filed January 12, 2001, which are incorporated by reference in their entirety.

### (a) Preparation of 15 nm diameter gold nanoparticles

Gold colloids (∼ 15 nm diameter) were prepared by reduction of HAuCl₄ with citrate as described in Frens, 1973, Nature Phys. Sci., 241:20-22 and Grabar, 1995, Anal. Chem.67:735*.* Briefly, all glassware was cleaned in aqua regia (3 parts HCl, 1 part HNO₃), rinsed with Nanopure H₂O, then oven dried prior to use. HAuCl₄ and sodium citrate were purchased from Aldrich Chemical Company. Aqueous HAuCl₄ (1 mM, 500 mL) was brought to reflux while stirring. Then, 38.8 mM sodium citrate (50 mL) was added quickly. The solution color changed from pale yellow to burgundy, and refluxing was continued for 15 min. After cooling to room temperature, the red solution was filtered through a Micron Separations Inc. 0.2 micron cellulose acetate filter. Au colloids were characterized by UV-vis spectroscopy using a Hewlett Packard 8452A diode array spectrophotometer and by Transmission Electron Microscopy (TEM) using a Hitachi 8100 transmission electron microscope.

### (b) Synthesis of steroid disulfide modified oligonucleotides (SDO)

Oligonucleotides corresponding to an aptamer sequence specific for IgE, APC gene DNA sequence, or MecA gene DNA sequence, or A₁₀ were synthesized on a 1 micromole scale using an Applied Biosystems Expedite 8909 DNA synthesizer in single column mode using phosphoramidite chemistry. Eckstein, F. (ed.) Oligonucleotides and Analogues: A Practical Approach (IRL Press, Oxford, 1991). All synthesis reagents were purchased from Glen Research or Applied Biosystems. Average coupling efficiency varied from 98 to 99.8%, and the final dimethoxytrityl (DMT) protecting group was removed from the oligonucleotides so that the steroid disulfide phosphoramidite could be coupled.

To generate 5'-terminal steroid-cyclic disulfide oligonucleotide derivatives (see Letsinger et al., 2000, Bioconjugate Chem. 11:289-291 and PCT/US01/01190 (Nanosphere, Inc.), the disclosure of which is incorporated by reference in its entirety), the final coupling reaction was carried out with a cyclic dithiane linked epiandrosterone phosphoramidite on Applied Biosystems automated Expedite 8909 synthesizer, a reagent that prepared using trans 1,2 -dithiane-4,5-diol, epiandrosterone and p-toluenesulphonic acid (PTSA) in presence of toluene. The phosphoramidite reagent may be prepared as follows: a solution of epiandrosterone (0.5g), trans 1,2-dithiane-4,5-diol (0.28 g), and p-toluenesulfonic acid (15 mg) in toluene (30 mL) was refluxed for 7 h under conditions for removal of water (Dean Stark apparatus); then the toluene was removed under reduced pressure and the reside taken up in ethyl acetate. This solution was washed with 5 % NaHCO₃, dried over sodium sulfate, and concentrated to a syrupy reside, which on standing overnight in pentane/ether afforded a steroid-dithioketal compound as a white solid (400 mg); Rf (TLC, silica plate, ether as eluent) 0.5; for comparison, Rf values for epiandrosterone and 1,2-dithiane-4,5-diol obtained under the same conditions are 0.4, and 0.3, respectively. The compound was purified by column chromatography. Subsequently, recrystallization from pentane/ether afforded a white powder, mp 110-112 °C; ¹H NMR, δ 3.6 (1H, C³OH), 3.54-3.39 (2H, m 2OCH of the dithiane ring), 3.2-3.0 (4H, m 2CH₂S), 2.1-0.7 (29H, m steroid H); mass spectrum (ES⁺) calcd for C₂₃H₃₆O₃S₂ (M+H) 425.2179, found 425.2151. Anal. (C₂₃H₃₆O₃S₂₎ S: calcd, 15.12; found, 15.26. To prepare the steroid-disulfide ketal phosphoramidite derivative, the steroid-dithioketal (100 mg) was dissolved in THF (3 mL) and cooled in a dry ice alcohol bath. N,N-diisopropylethylamine (80 µL) and β- cyanoethyl chlorodiisopropylphosphoramidite (80 µL) were added successively; then the mixture was warmed to room temperature, stirred for 2 h, mixed with ethyl acetate (100 mL), washed with 5% aq. NaHCO₃ and with water, dried over sodium sulfate, and concentrated to dryness. The residue was taken up in anhydrous acetonitrile and then dried under vacuum; yield 100 mg; ³¹P NMR 146.02. The epiandrosterone-disulfide linked oligonucleotides were synthesized on Applied Biosystems Expedite 8909 gene synthesizer without final DMT removal. After completion, epiandrosterone-disulfide linked oligonucleotides were deprotected from the support under aqueous ammonia conditions and purified on HPLC using Ion-exchange chromatography.

Ion-exchange HPLC was performed using the AKTA Basic HPLC system form Amersham Biosciences equipped with a 375mm x 16mm columm packed with Source Q Resin and a column heater set at 65°C. Using 20mM Na Acetate/10% CH₃CN/20mM NaCl₄ buffer and a gradient of 20mM NaAcetate/10% CH₃CN/ 600mM NaCL₄. The flow rate was at 5 ml/min. with UV detection at 260 nm. After collecting the peak of interest, the solution is passed through a membrane in order to remove excess salt. The solution was then evaporated to near dryness and reconstituted in 250mM phosphate buffer pH 7. The amount of oligonucleotide was determined by absorbance at 260 nm, and final purity assessed Ion -exchange chromatography.

### (c) Attachment of single stranded aptamer oligonucleotides to 15 nm diameter gold particles

A solution of ~13.75 nM gold nanoparticles (~ 15 nm diameter) was prepared using the citrate reduction method. The gold nanoparticle probes were prepared by loading the ~ 15 nm diameter gold particles (~13.75 nM) with steroid disulfide modified oligonucleotides using a modification of previously developed procedures. See, for instance, U.S. Patent no. 6,767,702, which is incorporated by reference in its entirety.. For the aptamer oligonucleotide conjugates, 3 nmol of aptamer oligonucleotide and 1 nmol of A₁₀ was added per 1 mL of 13.7 nM gold nanoparticle and incubated for 15 hours at room temperature. The solution was raised to 0.1 M NaCl, 10 mM phosphate (pH 7), 0.01% azide using 1 M NaCl, 100 mM phosphate (pH 7), 1% azide and incubated for 12 hours. The solution was then raised to 0.3 M NaCl using 5M NaCl buffer and incubated for an additional 12 hours. The solution was then raised to 0.8 M NaCl using the same buffer and incubated for an additional 24 hours. The aptamer-gold nanoparticle conjugates were isolated with a Beckman Coulter Microfuge 18 by centrifugation at 13000 rpm for 20 minutes. After centrifugation, the supernatant was removed, and the dark red gelatinous residue remaining at the bottom of the eppendorf tube was redispersed in water. This step was repeated twice to ensure removal of all unbound oligonucleotide. The final nanoparticle concentration was adjusted to 10 nM based on UV-visible absorbance at 520 nm using an estimated extinction coefficient of ε₅₂₀ = 2.4 x 10⁸ M⁻¹cm⁻¹.

The following aptamer-oligonucleotide conjugates specific for human IgE were prepared in this manner:
IgE Probe: gold-S'-5'-[gcgcggggcacgtttatccgtccctcctagtggcgtgccccgcgc-3']ₙ (SEQ ID NO: 1)

The following oligonucleotide-gold nanoparticle conjugates were prepared under the same conditions and procedure without A₁₀ diluent (4 nmol of the oligonucleotide used).
MECA 2Q: gold-S'-5'-[a₁₅-peg-atggcatgagtaacgaata]ₙ (SEQ ID NO: 2)
CRC 4D: gold-S'-5'-[a₂₀-gcagaataaaag]ₙ (SEQ ID NO: 3)
S' indicates a connecting unit prepared via an epiandrosterone disulfide group; n indicates that a number of oligonucleotides are attached to each gold nanoparticle.

### (d) Preparation of gold nanoparticle- antibody conjugates

Affinity purified Anti-IgE polyclonal antibody was purchased from Chemicon international. The antibody was conjugated to 15 nm diameter gold nanoparticles using procedures previously described by British BioCell International (BBI). Briefly, the 15 nm diameter gold particles were adjusted to a pH between 9-10 using sodium carbonate buffer. 5 µg/mL of antibody was added to the gold nanoparticle and incubated at room temperature for 1.0 hours. Next, BSA was added to the solution to stabilize the particles. The antibody-gold nanoparticle conjugates were isolated with a Beckman Coulter Microfuge 18 by centrifugation at 13000 rpm for 20 minutes. After centrifugation, the supernatant was removed, and the dark red gelatinous residue remaining at the bottom of the eppendorf tube was redispersed in buffer (0.2 M Tris buffer (pH 8.5), 0.1 % BSA and 0.01% azide). This step was repeated to ensure removal of all unbound antibody. The final nanoparticle concentration was adjusted to 10 nM based on UV-visible absorbance at 520 nm using an estimated extinction coefficient of ε₅₂₀ = 2.4 x 10⁸ M⁻¹cm⁻¹.

### (e) Preparation ofprotein microarrays

Purified Human IgE was purchased from Chemicon International. Bovine Serum Albumin (BSA) and IgG were purchased from Sigma Aldrich. The proteins were arrayed onto Codelink slides (Amersham, Inc.) using a GMS417 arrayer (Affymetrix). The human IgE, IgG, and BSA were arrayed in 150mM phosphate buffer (pH 8.5) at a concentration of 2 mg/mL. The slides were incubated overnight in a humidity chamber, and subsequently washed with TBS-T Buffer (150mM NaCl/ 10mM Tris Base buffer (pH 8) containing 0.05 % Tween. All of the proteins were arrayed in triplicate. The position of the arrayed spots was designed to allow multiple hybridization experiments on each slide, achieved by partitioning the slide into separate test wells by silicon gaskets (Grace Biolabs).

### (ƒ) Specific Detection of IgE

Experimental: Binding of the IgE specific aptamer oligonucleotide - gold nanoparticle conjugate detection probes (SEQ ID NO: 1) or other oligonucleotide - gold nanoparticle conjugate detection probes used as negative controls (SEQ ID NO: 2 and 3) was tested using microarrayer glass slides with immobilized IgE, as well negative control spots of IgG and BSA, Figure 2. Each 50 µL binding reaction contained - 2 nM of detection probes in binding buffer consisting of 1x phosphate buffer saline (pH 7), 1.0 mM MgCl₂. The aptamer coated gold probe solutions were heated to 95°C for three minutes in an eppendorf tube, cooled to room temperature for five minutes, or put on ice for 2 minutes and then pipetted onto the protein array and incubated for one hour at room temperature (~ 24°C). Post-hybridization washes were initiated by immersing the glass slide in 1x phosphate buffer saline (pH 7), 1.0 mM MgCl₂, 0.001% Tween20 for 1 minute, followed by dipping the slide into water (~ 2 seconds) to remove excess salts from the glass slide surface. The slides were stained with silver enhancing solution using a previously described silver amplification method (Taton et. al, 2000, Science, Vol. 289, 1757-1760), dried on a spin dryer, and imaged on an ArrayWorx® biochip reader (Model no. AWE, Applied Precision Inc., Issaquah, WA, U.S.A.) (Figures 3 and 4). Scatter signals from each spot on the array were quantified using ArrayWorx software. As a positive control binding reaction, Anti-IgE antibody - gold nanoparticle conjugate detection probes were tested. The protocol and buffer used in the binding reaction was the same as described above for the aptamer coated gold probes, with the omission of the initial heating step.

Results: Binding of the IgE specific aptamer coated gold probes (AGPs) was compared to Anti-IgE antibody coated gold probes (Ab-GPs) in a binding reaction to IgE immobilized on a glass surface (see experimental). The glass slides were imaged after silver amplification, and the net signal was quantified on the array (Figure 3). The AGPs (SEQ ID NO: 1) exhibited specific binding to IgE on the array based on the net signal intensity from the positive (IgE) and negative control (IgG, BSA) spots immobilized on the glass slide. Specific binding to IgE was also observed in a positive control reaction performed with the Ab-GPs at a similar concentration of probe. The IgE specific AGPs also were compared to other DNA-modified gold nanoparticles (SEQ ID No's: 2 and 3) using the silver amplification assay (Figure 4). The DNA-modified gold nanoparticles did not bind to IgE or the negative controls, whereas the AGPs specifically bound to IgE. These experiments clearly demonstrated that the aptamers were functional on the gold nanoparticle surface enabling specific detection of proteins. This system can be extended to the detection of proteins in a sandwich type assay using silver amplification as well as a colorimetrically by 'sandwiching' a protein target with two aptamer coated gold probes.

### Example 2. Methods for the preparation of oligonucleotide aptamer derivatized gold nanoparticles and the detection of human IgE (Method no. 2)

For additional proof of concept studies, this Example evaluates a well studied DNA aptamer sequence(Wiegand, T. W.; Williams, P. B.; Dreskin, S. C.; Jouvin, M. H.; Kinet, J. P.; Tasset, D. J. Immunol. (1996), 157, 221-230; Liss, M.; Petersen, B.; Wolf, H.; Prohaska, E. Anal. Chem. (2002), 74, 4488-4495) which has a high binding affinity for human IgE:
(5' cgcggggcacgtttatccgtccctcctagtggcgtgccccgcgc 3') [SEQ ID NO. 4]
The anti-IgE aptamer forms a stem loop structure that binds to the F_{c} region of the IgE target with a measured K_{d} of 8.4 nM. (see Liss, M.; Petersen, B.; Wolf, H.; Prohaska, E. Anal. Chem. (2002), 74, 4488-4495). The anti-IgE aptamer was conjugated to 15 nm diameter gold particles via a thiol modification using the salt aging procedure discussed above (Storhoff, J. J.; Marla, S. M.; Bao, P.; Hagenow, S.; Mehta, H.; Lucas, A.; Garimella, V.; Patno, T.; Buckingham, W.; Cork, W.; Muller, U. Biosens. Bioelectron. (2004), 19, 875-883). A negative control DNA sequence was conjugated to 15 nm diameter gold particles for comparison:
Control 1: 5' aaaaaaaaaaaaaaaatggcatgagtaacgaata 3' [SEQ ID NO. 5]

Test arrays were fabricated by covalently immobilizing the target protein (human IgE antibodies) and control protein (human IgG antibodies) onto glass slides containing amine reactive groups using a contact printing robotGMS417 arrayer (Affymetrix). Anti-IgE AGP binding studies were conducted on the test array using a previously described silver amplification and imaging procedure (Figure 5A). In a typical experiment, the anti-IgE AGPs (2 nM, 50 µL) were incubated on the IgE coated glass slides in 1X PBS buffer (pH 7.2), 1 mM MgCl₂ for one hour at ~ 24 °C. After washing the slides in a similar buffer containing 0.001 % Tween20, the slides were stained with silver enhancing solution for five minutes (Taton, T. A.; Mirkin, C. A.; Letsinger, R. L. Science (2000), 289, 1757-1760. Storhoff, J. J.; Marla, S. M.; Bao, P.; Hagenow, S.; Mehta, H.; Lucas, A.; Garimella, V.; Patno, T.; Buckingham, W.; Cork, W.; Muller, U. Biosens. Bioelectron. (2004), 19, 875-883 ). This procedure was repeated on a separate array using the negative control DNA modified gold particles. The light scattered by silver amplified gold particles captured on the slide was analyzed with a commercial scanner that illuminates the glass slide with white light and records an image with a CCD camera (Storhoff, J. J.; Maria, S. M.; Bao, P.; Hagenow, S.; Mehta, H.; Lucas, A.; Garimella, V.; Patno, T.; Buckingham, W.; Cork, W.; Muller, U. Biosens. Bioelectron. (2004), 19, 875-883). The image and corresponding data analysis demonstrate that the anti-IgE AGPs bind specifically to the IgE antibodies immobilized on the array, while the negative control DNA-modified gold probes exhibit little/no signal at the IgE antibody test sites (Figure 5B). These data demonstrate that the anti-IgE aptamer sequence is functional after attachment to the gold particle surface, and the aptamer binds specifically to the immobilized IgE target. Additionally, these data provide preliminary evidence that DNA-modified gold particles have low non-specific binding to the antibodies immobilized on the slides. This is consistent with previous studies on DNA microarrays (Taton, T. A.; Mirkin, C. A.; Letsinger, R. L. Science (2000), 289, 1757-1760).

Affinity purified goat anti-human IgE polyclonal antibody and purified Human IgE were purchased from Chemicon International. Human IgG was purchased from Sigma Aldrich. CodeLink slides were purchased from Amersham, Inc. 60 nm diameter gold particles were purchased from British BioCell International (BBI). HAuCl₄·3H₂O, trisodium citrate, Tween 20, sodium dodecyl sulfate (SDS), and Silver enhancer solution A and B were purchased from Sigma Aldrich Chemical company.

### (a) Preparation of 15 mn diameter gold particles.

Gold colloids (~ 15 nm diameter) were prepared by reduction of HAuCl₄ with citrate as described in Frens, 1973, Nature Phys. Sci., 241:20-22 and Grabar, 1995, Anal. Chem.67:735. Briefly, all glassware was cleaned in aqua regia (3 parts HCl, 1 part HNO₃), rinsed with Nanopure H₂O, then oven dried prior to use. HAuCl₄ and sodium citrate were purchased from Aldrich Chemical Company. Aqueous HAuCl₄ (1 mM, 500 mL) was brought to reflux while stirring followed by the rapid addition of 38.8 mM sodium citrate (50 mL). The solution color changed from pale yellow to burgundy, and refluxing was continued for 15 min. After cooling to room temperature, the red solution was filtered through a Micron Separations Inc. 0.2 micron cellulose acetate filter. Au colloids were characterized by UV-vis spectroscopy using a Hewlett Packard 8452A diode array spectrophotometer.

### (b) Reparation of DNA-modified gold nanoparticles.

The as prepared gold nanoparticles were derivatized with thiol functionalized oligonucleotides using a previously described salt aging protocol (Storhoff, J. J.; Maria, S. M.; Bao, P.; Hagenow, S.; Mehta, H.; Lucas, A.; Garimella, V.; Patno, T.; Buckingham, W.; Cork, W.; Muller, U. Biosens. Bioelectron. (2004), 19, 875-883, and references therein).. Briefly, the IgE aptamer (3 µM final concentration) and A₂₀ diluent sequence (1 µM final concentration) are initially incubated with the as prepared gold nanoparticles for > 16 hours, followed by successive additions of phosphate buffered saline to a final concentration of 0.8 M NaCl, 10 mM phosphate (pH 7). After standing for > 10 hours, the probes were isolated by centrifugation, washed in an equivalent amount of water, and then redispersed in 0.1 M PBS, 0.01% azide at a particle concentration of 10 nM. All probes were stored at 4 °C.

The 60 nm diameter gold nanoparticle conjugates are prepared using a similar procedure with the following modifications. First, 0.9uM of aptamer oligonucleotide and 1.8uM of A₂₀ diluent sequence are added to the particles obtained from BBI. Next, sodium dodecyl sulfate (SDS) detergent is added to a final concentration of 0.01%, followed by successive additions of NaCl to a final concentration of 0.8 M NaCl (Storhoff, J. J.; Lucas, A. D.; Viswanadham, G.; Bao, Y. P.; Muller, U. Nat. Biotechnol. (2004), 22, 883-887, and references therein). The aptamer-modified particles are isolated by centrifugation (2300 rcf for 30 minutes), washed in an equivalent amount of water, and then redispersed in 10mM Sodium Phosphate, 0.1 M NaCl, 0.01% azide.

### (c) Preparation of antibody-modified gold nanoparticles.

Polyclonal antibodies were conjugated to 60 nm diameter gold nanoparticles using procedures previously described by British BioCell International (BBI) that accompany the purchase of gold colloid. Briefly, the 60 nm diameter gold particles were adjusted to a pH between 9-10 using sodium carbonate (~ 25 uL of 0.1 M sodium carbonate added to 1 ml of as received colloid). 3 µg of antibody was added per 1 mL of gold nanoparticle and incubated at room temperature for 1.0 hours. Next, BSA was added to a final concentration of 1% to stabilize the particles. The antibody-gold nanoparticle conjugates were isolated with a Beckman Coulter Microfuge 18 by centrifugation at 2100 rcf for 25 minutes. After centrifugation, the supernatant was removed, and the dark red gelatinous residue remaining at the bottom of the eppendorf tube was redispersed in buffer (20 mM Tris buffer (pH 8.5), 0.5 % BSA and 0.01% azide). The final nanoparticle concentration was calculated based on the UV-visible absorbance reading at 520 nm using an extinction coefficient of ε₅₂₀ = 2.8 x 10¹⁰ M⁻¹cm⁻¹.

### (d) Preparation of arrays of immobilized antibodies.

The human IgE, anti-IgE and IgG antibodies were arrayed onto Codelink slides (Amersham, Inc.) using an Affymetrix GMS 417 pin and ring microarrayer equipped with a 500 micron diameter pin. Typically, the antibodies were buffered in 1X PBS pH 7.2, 60mM Trehalose, at a final concentration of 500 ug/mL. After printing, the slides were incubated overnight in a humidity chamber, and subsequently washed with TBS-T Buffer (150mM NaCl/ 10mM Tris Base buffer (pH 8) containing 0.05 % Tween20). The antibodies were arrayed in triplicate, and ten replicates of the arrayed spots were produces on each slide. The arrays were partitioned into separate test wells using silicone gaskets (Grace Biolabs).

### (e) Assays and Imaging.

Assays were performed using silicone gaskets at a 40 - 50 uL volume. After completion of the assay, the gaskets are removed and the slides are centrifuged to remove excess liquid. For silver development, silver enhancer solutions A and B were mixed 1:1 and placed onto each slide and incubated for 5 minutes. Subsequently, the slides were washed with water and imaged using a commercial image analysis system which illuminates the slide with white light using a metal halide arc lamp and collects the scattered light from the illuminated slide through a microscope objective onto a cooled CCD camera (Storhoff, J. J.; Marla, S. M.; Bao, P.; Hagenow, S.; Mehta, H.; Lucas, A.; Garimella, V.; Patno, T.; Buckingham, W.; Cork, W.; Muller, U. Biosens. Bioelectron. (2004), 19, 875-883, and references therein)..

### Example 3: Comparison of Nanoparticle-aptamer conjugates and Nanoparticle-antibody conjugates

In this Example, anti-IgE antibody gold nanoparticle conjugates were prepared for comparison to the anti-IgE AGPs as detection labels for binding to IgE target in a sandwich assay format (Figure 5). For these studies, goat polyclonal antibodies developed against human IgE were passively adsorbed to 60 nm diameter gold particles using well established procedures, and the anti-IgE aptamer was conjugated to 60 nm diameter gold particles (see Example 2 above). The 60 nm diameter gold particles were selected to demonstrate that AGPs of different sizes can be prepared and used as detection labels. Metal particles > 40 nm diameter scatter light of a specific color at the surface plasmon resonance frequency (Yguerabide, J.; Yguerabide, E. E. Anal. Biochem. (1998), 262, 157-176) and can be used for multicolor labeling on arrays by controlling particle size, shape, and chemical composition (Taton, T. A.; Lu, G.; Mirkin, C. A. J. Am. Chem. Soc. (2001), 123, 5164-5165; Jin, R. C.; Cao, Y. W.; Mirkin, C. A.; Kelly, K. L.; Schatz, G. C.; Zheng, J. G. Science (2001), 294, 1901-1903) as well as homogeneous detection assays (Storhoff, J. J.;, Lucas, A. D.; Viswanadham, G.; Bao, Y. P.; Muller, U. Nat. Biotechnol. (2004), 22, 883-887). Test arrays were fabricated by immobilizing the polyclonal anti-IgE antibodies and human IgG antibodies (not specific for the target) onto glass slides as previously described.

In the first step of the assay, different concentrations of human IgE target (1 ng/mL - 1 ug/mL) or a no target control containing 1 ug/mL human IgG were incubated on separate test arrays in 1X PBS buffer (pH 7.2), 1 mM MgCl₂, 0.01 % Tween 20 for 7 minutes at ~ 24°C. After removing the target solution from the array, the slides were reacted with the anti-IgE antibody coated gold probe (400 pM) or the anti-IgE AGP (400 µM) in the same buffer containing 2 % dextran sulfate for 3 minutes, followed by a buffer wash, and imaging as described above. The most notable difference between the antibody and aptamer coated gold probe is the binding specificity. The arrays labeled with antibody probes exhibited a substantially higher background signal in the no target control well when compared to the arrays labeled with aptamer probes (Figure 7). This was attributed to non-specific binding of the antibody probes to both the goat polyclonal anti-IgE and human IgG control antibodies attached to the glass slide. As a result, 1 ng/mL of human IgE target was detectable using the aptamer probes based on a net signal that was greater than 3 standard deviations above the no target control, while only 10 ng/mL was detectable above this threshold using the antibody probes. The non-specific binding of the antibody probes is alleviated by removing the dextran sulfate or lowering the probe concentration, but these changes do not improve the limit of detection in the assay.

In conclusion, Examples 1 -3 demonstrate that DNA-based aptamers can be conjugated to gold particles of various sizes (15 - 60 nm diameter) and used as detection labels for protein targets. In a sandwich assay performed on antibody arrays, the AGPs improved the limit of detection by ~ 1 order of magnitude when compared to antibody coated gold probes. This is attributed to lower non-specific binding/cross reactivity with the antibodies attached to the array. Furthermore, these probes are stable to prolonged storage and to heat and saline as noted in previous studies on DNA-modified gold particles. The novel labeling technology discussed herein can be applied to other nanoparticle-based detection methodologies used for immunoassays or antibody array (Grubisha, D. S.; Lipert, R. J.; Park, H.-Y.; Driskell, J.; Porter, M. D. Anal. Chem. 2003, 75, 5936-5943; Storhoff, J. J.; Lucas, A. D.; Viswanadham, G.; Bao, Y. P.; Muller, U. Nat. Biotechnol. 2004, 22, 883-887; Cao, Y. W. C.; Jin, R. C.; Mirkin, C. A. Science 2002, 297, 1536-1540), as well as other molecules of interest for which aptamers can be designed (Jayasena, S. D. Clin. Chem. 1999, 45, 1628-1650).

### Example 4: Evaluation of Surface density of Nanoparticle-aptamer probes

In this Example, nanoparticles labeled with aptamers of varying surface density were prepared and evaluated. These labeled nanoparticles were prepared in accordance with the procedures described in Example 1 The number of available aptamers on IgE aptamer coated 60 nm diameter gold particles (A10-aptamer 1or T10-aptamer 1 probe sequence) with varying ratios of A₂₀ diluent were measured using a fluorescence-based assay previously reported (Demers et. al, Anal. Chem., 2000), Figure 8. The gold particles tested in the first experiment were loaded with ratios of IgE aptamer:A₂₀ diluent ranging from 100:0, 2:1, 1:2, 1:8, or 0:100 A₂₀ (control), or with a T10-aptamer. The probe sequences are shown in Table 1 below.

**Table 1. Sequence table for reference.**

| | Probe sequences for gold particle |
|---|---|
| **T10 - Aptamer 1** | 5' ttt ttt ttt tgcg cgg ggc acg ttt atc cgt ccc tcc tag tgg cgt gcc ccg cgc 3' [SEQ ID NO. 8] |
| **A10 - Aptamer 1** | 5' aaa aaa aaa agcg cgg ggc acg ttt atc cgt ccc tcc tag tgg cgt gcc ccg cgc 3' [SEQ ID NO. 9] |
| **A20 diluent** | 5' aaa aaa aaa aaa aaa aaa aa 3' [SEQ ID NO. 7] |

The number of available aptamers for each aptamer coated gold probe was measured on two separate probe preparations. The number of available aptamers per gold particle is dependent on the ratio of aptamer:A₂₀ loaded onto the particle, Table 2.

**Table 2. Number of available aptamers/gold particle as a function of particle loading conditions.**

| **IgE Aptamer. A₂₀ Diluent Ratio** | **Number of aptamers/particle (run 1)** | **Number of aptamers/particle (run 2)** | **Average Number of Aptamers/particle (aptamers per cm²)** |
|---|---|---|---|
| 100:0 | 514± 60 | 514 | 4.54 x 10¹² |
| 2:1 | 338 ± 27 | 428 | 3.39 x 10¹² |
| 1:2 | 220 ± 31 | 210 | 1.90 x 10¹² |
| 1:8 | Not performed | 101 | 8.93 x 10¹¹ |
| T10 Aptamer (100:0) | 724 ± 103 | Not performed | 6.40 x 10¹² |
| Control (All diluent) | 3.3 ± 1.7 | 0.1 | 1.50 x 10¹⁰ |

Aptamer coated gold probes with a varying number of available aptamers and linker sequence (A20 or T20) were tested for binding to IgE target to determine optimal aptamer loading. Anti-IgE antibody was printed onto the functionalized glass slide to capture the IgE target. A serial dilution of anti-IgE antibody and IgG control antibodies (1000, 500, and 250 ug/mL) was arrayed in triplicate on the functionalized glass slides. Next, the IgE target (1 ug/mL) was incubated on separate test arrays for 30 minutes. The IgE target was removed from the arrays, and the aptamer coated gold probes (see Table 2) were incubated on separate arrays in optimized binding buffer for 15 minutes. The total scattering intensity from each anti-IgE and IgG spot located on the test slides was measured to determine the amount of specifically bound AGP, Figure 9. For comparison, a polyclonal anti-IgE probe also was tested in a separate reaction, along with a gold probe coated with A₂₀ as a negative control. Each of the aptamer coated gold probes as well as the anti-IgE antibody coated gold probes bind the IgE target specifically based on a comparison of the total scattering intensity from anti-IgE and IgG control spots. The control reactions without IgE target exhibit minimal scattering signal indicating the AGP binding is IgE specific. Quantitatively, the preliminary data suggest the aptamer coated gold probes exhibit more binding to the IgE target than the anti-IgE antibody probes, regardless of the number of the available aptamers, under these binding conditions.

The binding kinetics of each aptamer coated gold probe was tested by incubation at a defined probe concentration on a test array with immobilized IgE for a defined period of time (3-15 minutes). The gold probe scatter signal from IgE target bound to anti-IgE test sites printed at 250, 500, or 1000 ug/mL (IgG test sites serve as a negative control) was quantitated for each probe reaction. At the shortest probe incubation time of 3 minutes, the probe with the fewest aptamers (1:8 - 101 aptamers/particle) exhibited much less signal than the other three probes, Figure 10. The highest signal on average was obtained from the AGPs loaded with 420 (2:1) or 228 (1:2) aptamers, with a nearly equivalent average signal from the AGP loaded with 514 aptamers (FL). At a 7 minute probe incubation time, a similar trend was observed, while at 15 minutes the 1:8 probe was nearly equivalent to the other AGPs in terms of signal intensity. These data suggest that aptamer coated gold probes with > 228 aptamers exhibit significantly faster binding kinetics than probes with < 101 aptamers/particle. In addition, it suggests that probes with > 228 aptamers exhibit similar binding kinetics under these conditions, suggesting that a range of aptamer surface coverages may be suitable for a two step assay once a minimum number of aptamers/particle is reached.

### Example 5: Preparation of Aptamer-Coated Gold Probe Arrays

In this Example, the preparation of aptamer-coated gold probe arrays is described. The aptamer coated gold probes were immobilized onto a waveguide substrate through hybridization to an amine modified T₂₀ oligonucleotide (SEQ ID NO: 10) covalently attached to the surface (Figure 11). Typically, the amine modified T₂₀ oligonucleotides were resuspended in 1X PBS pH 7.2 at a final concentration of 500 uM and arrayed onto Codelink slides (Amersham, Inc.) or Superaldehyde slides (Telechem International) using an Affymetrix GMS 417 pin and ring microarrayer equipped with a 500 micron diameter pin. The slides were incubated overnight in a humidity chamber and subsequently washed with 1X PBS (pH 7.2), 0.01% Tween 20 buffer. Typically, the oligonucleotides were arrayed in triplicate in two rows, and ten replicates of the arrayed spots were produced on each slide. The arrays were partitioned into separate test wells using silicone gaskets (Grace Biolabs). The aptamer-modifed gold probes (sequence A10-aptamer with an T₂₀ diluent, SEQ ID NO: 10) were then added at various concentrations in a second step for 15 minutes at room temperature (1X PBS, 1 mM MgGl₂, 0.01% Tween20) to form the 'aptamer coated gold probe arrays'. Each probe array was illuminated with white light and then imaged with a color CCD camera to record the scatter color. As shown in Figure 12, the AGPs were immobilized onto the Aldehyde substrate at the complementary T₂₀ spots, and the probes scatter predominantly green - greenish/yellow light depending on the probe concentration. This indicates that the density of AGP bound to the surface can be controlled by the amount of gold probe added.

### Example 6: Human IgE Detection on anti-IgE Aptamers coated Gold Probe Arrays

In this Example, the detection of human IgE target was tested on the anti-IgE aptamer coated gold probe arrays prepared in Example 5. See Figure 13. For these studies, the anti-IgE aptamer coated gold probes (75 pM) were immobilized on the T₂₀ arrays as describe above. All assay steps were performed at room temperature in 40 µL reaction volumes. In the first step, different concentrations of human IgE (2 ug/mL - 1 ng/mL) or human IgG as a negative control (2 ug/mL) were incubated on separate test arrays for 30 minutes in 1 mM MgCl₂, 1X PBS, 0.01% Tween20. In the second step, anti-IgE antibody coated gold probes (prepared as described above) were incubated on the array for 10 minutes at a probe concentration of 450 pM in a buffer containing 1 mM MgCl₂, 1X PBS, 0.01% Tween20, 2 % dextran sulfate. The scatter color from each probe array was recorded using a color CCD camera after illumination with white light (Figure 14). A change in scatter color from green to orange was observed for samples containing > 50 ng/mL of IgE target (2.5 ng total IgE). Samples containing < 10 ng/mL of IgE target or 2 µg/mL of IgG target remained green in scatter color. It should be noted that the scatter color can also be detected visually with the naked eye.

In an alternative method of analysis, the slide was washed in a 5% formamide, 1% Tween 20 prior to imaging with the Verigene ID detection system (Figure 15). The washing step reduced the amount of green scatter from the human IgG control sample while increasing the colorimetric red-shift in scatter observed for the human IgE target samples. This indicated that the aptamer coated gold probes may be removed from the array by dehybridization in the wash step while AGP probe complexes formed from human IgE target and anti-IgE antibody coated gold probe remain attached to the slide. Therefore, background signal due to unbound probes (green scatter) may be selectively removed via a simple washing process. It should be noted that this effect was first observed after washing the slides with water, and subsequent experiments using different concentrations of formamide and tween demonstrated that 5% formamide, 1% Tween 20 produced the best removal of background while retaining signal from gold probe complexes. The net signal intensity from a 10 ng/mL sample of human IgE was > 3 standard deviations over the net signal intensity of a human IgG negative control sample. This represented at least a 5 fold improvement in detection limit over visually analyzing the color change (~ 50 ng/mL limit of detection), and it demonstrated that the AGP arrays can be imaged with the Verigene ID detection system in conjunction with a wash step.

### Example 7. Aptamers with a polyA tail: hybridization to a substrate and uses in detection

An anti-IgE aptamer with an A20 tail [SEQ ID NO: 10] was synthesized by standard phosphoramidite chemistry. Amine modified T20 and A20 oligonucleotides were synthesized by phosphoramidite chemistry and redispersed in 10 mM phosphate (pH 8) at a final concentration of ~ 0.1 - 1 mM. The amine modified oligonucleotides were attached to Codelink substrates using a pin and ring microarrayer equipped with a 500 um diameter pin following the manufacturer's recommendations for attachment of oligonucleotides.
5' aaa aaa aaa a aaa aaa aaa agcg egg ggc acg ttt atc cgt ccc tcc tag tgg cgt gcc ccg cgc 3' [SEQ ID NO. 10]
The A20 tailed anti-IgE aptamer (SEQ ID NO: 10) was hybridized to the T20 oligonucleotide attached to the substrate at a concentration of 10 nM for 15 minutes at room temperature in 1X PBS, 1 mM MgCl2, 0.01 % Tween20 buffer (referred to as incubation buffer). The slide was washed with ~ 100 mL of incubation buffer. Next, human IgE target or human IgG negative control (50 pg/mL - 500 ng/mL) in incubation buffer was added to the substrate for 7 minutes. Subsequently, the target solutions were removed from the slide, and 400 pM of polyclonal goat x-IgE coated 60 nm diameter gold probes in incubation buffer with 2% dextran sulfate was added to each array and incubated for three minutes. The slide was washed with ~ 100 mL of incubation buffer, and imaged on an ArrayWorx image analyzer. See Figure 19 which illustrates detection of IgE using an xIgE aptamer tailed with A20 hybridized to T20 on the array.

## Claims

1. A method for detecting at least one typ of target analyte in a sample, the target analyte having at least two binding sites, the method comprising the steps of:
a) providing at least one type of nanoparticle probe comprising detector aptamers, wherein the detector aptamers on each type of probe have a configuration that can bind to a first binding site of a specific type of target analyte;
b) contacting the sample with the nanoparticle probe under conditions that are effective for the binding of the detector aptamers to the target analyte; and
c) detecting whether the detector aptamers binds to the target analyte,
wherein the aptamers are present on the nanoparticles at a surface density ranging from between 8.9 x 10¹¹ and 6.4 x 10¹² aptamers/cm².

2. The method of claim 1, the method further comprising in step
a) providing a substrate having at least one type of capture probe bound thereto, wherein the capture probe can bind to a second binding site of a specific target analyte; and further comprising in step b) contacting the sample with the substrate under conditions that are effective for the binding of the capture probe to the second binding site of the target analyte to form a complex of target analyte bound to said probe and substrates; and detecting in step c for the presence or absence of the complex, wherein the presence or absence of the complex is indicative of the presence or absence of the specific target analyte in the same.

3. The method of claim 1, the method comprising in step a) aptamers capable of binding to two or more binding sites of the target analyte

4. The method of claim 1, the method comprising in step
a) at least two types of nanoparticle probes having detector aptamers bound thereto, each type of aptamer capable of binding to a different binding site of the target analyte; and in step
b) contacting the sample and the at least two types of nanoparticle probes having aptamers bound thereto under conditions effective to allow binding between the target analyte and the aptamers bound to the nanoparticles.

5. The method of claim 1, the method further comprising in step
a) providing at least one type of nanoparticles having antibodies bound there to, the antibodies capable of binding to a second binding site of the target analyte; and further comprising in step
b) contacting said sample, and the at least one type of nanoparticles having antibodies bound thereto under conditions effective to allow binding between the target analyte and the antibodies bound to the nanoparticles; and in step c) detecting for the presence or absence of the complex, wherein the presence or absence of the complex is indicative of the presence or absence of the specific target analyte in the same.

6. The method of claim 1, 2, 3, or 4 wherein in step a) at least one type of detector aptamer having an oligonucleotide tail is provided and a second oligonucleotide having a sequence that is complementary to at least a portion of a sequence of the oligonucleotide tail, said second oligonucleotide having an optional label is provided.

7. The method according to claim 2 or 6, wherein the capture probe comprises an antibody or a capture aptamer.

8. The method according to any one of claims 2, and 7, wherein the binding sites are epitopes that a specific capture probe binds to.

9. The method according to any one of claims 2, 6, 7 and 8, wherein two or more types of nanoparticle or aptamer probes are provided, each type of probes having detector aptamers bound thereto that are capable of binding to a different epitope on the same target analyte or to different target analytes, or both.

10. The method according to any one of claims 2, 6 and 7 to 9, wherein
i) the sample is first contacted with the nanoparticle or aptamer probe so that a target analyte present in the sample binds to the detector aptamers on the probe, and the target analyte bound to the nanoparticle or aptamer probe is then contacted with the substrate so that the target analyte binds to the capture probe on the substrate;
ii) the sample is first contacted with the substrate so that a target analyte present in the sample binds to a capture probe, and the target analyte bound to the capture aptamer is then contacted with the nanoparticle or aptamer probe so that the target analyte binds to the detector aptamers on the nanoparticle or aptamer probe; or
iii) the sample, the nanoparticle or aptamer probe and the capture probe on the substrate are contacted simultaneously.

11. The method according to any one of claims 2, 6 and 7 to 10, wherein the captured target-nanoparticle or -aptamer probe complex is detected by photonic, electronic, acoustic, opto-acoustic, gravity, electro-chemical, electro-optic, mass-spectrometric, enzymatic, chemical, biochemical, or physical means.

12. The method according to any one of claims 1 to 5, wherein the detection is by photonic, electronic, acoustic, opto-acoustic, gravity, electro-chemical, electro-optic, mass-spectrometric, enzymatic, chemical, biochemical, or physical means.

13. The method according to any one of claims 2, 7 to 10 and 12, wherein the nanoparticles are made of a noble metal, preferably of gold or silver, more preferably of gold.

14. The method according to any one of claims 2, 6 and 7 to 11, wherein the substrate
α) is a magnetic bead;
β) has a planar surface; or
γ) is made of glass, quartz, ceramic, or plastic.

15. The method according to any one of claims 1 to 6 and 7 to 13, wherein the detecting comprises
1) contacting the substrate with silver stain;
2) detecting light scattered by the nanoparticle; or
3) observation with an optical scanner.

16. The method according to claim 15, wherein the scanner is linked to a computer loaded with software capable of calculating grayscale measurements, and the grayscale measurements are calculated to provide a quantitative measure of the amount of analyte detected.

17. The method according to any one of claims 2, 6, 7 to 11, 15 and 16, wherein the substrate is addressable.

18. The method according to claim 17, wherein a plurality of capture probes, each of which can recognize a different target analyte, are attached to the substrate in an array of spots.

19. The method according to claim 18, wherein each spot of capture probes is located between two electrodes, the optional label on the aptamer probe is
a) nanoparticle(s) (are) made of a material that is a conductor of electricity, and step d) comprises detecting a change in conductivity.

20. The method according to claim 19, wherein the electrodes are made of gold and the nanoparticles are made of gold.

21. The method according to claim 19, wherein the substrate is contacted with silver stain to produce the change in conductivity.

22. A nanoparticle-aptamer conjugate probe comprising:
a) nanoparticles; and
b) at least one type of aptamer, the aptamers being present on the nanoparticles at a surface density ranging from between 8.9x10¹¹ and 6.4x10¹² aptamers/cm².

23. The probe according to claim 22 comprising at least two types of aptamers.

24. The probe according to any one of claims 22 to 23, wherein the nanoparticle further comprises oligonucleotides attached thereto in addition to the aptamers.

25. The probe according to claim 24, wherein the oligonucleotides are polyadenosine oligonudeotides, preferably A₁₀ or A₂₀.

26. The probe according to any one of claims 22 to 25, wherein the aptamers comprise phosphorothioate or phosphorodithioate moieties.

27. The probe according to any one of claims 22 to 26, wherein the nanoparticles are metallic nanoparticles or semiconductor nanoparticles.

28. The probe according to claim 27, wherein the nanoparticles are made of noble metal, preferably of gold.

29. A kit for detecting for one or more analytes in a sample, the kit comprising the aptamer detection probes according to any one of claims 22 to 28 and an optional substrate.

30. The kit according to claim 29 wherein the substrate is arrayed with at least one capture probe for a specific analyte.

## Patentansprüche

1. Verfahren zur Detektion von zumindest einer Art eines Zielanalyts in einer Probe, wobei der Zielanalyt zumindest zwei Bindungsstellen aufweist und das Verfahren die Schritte umfasst:
a) Bereitstellen von zumindest einer Art einer Nanopartikelsonde, umfassend Detektor-Aptamere, wobei die Detektor-Aptamere auf jeder Art von Sonde eine Konfiguration aufweisen, die an eine erste Bindungsstelle einer spezifischen Art eines Zielanalyts binden kann;
b) In Kontakt bringen der Probe mit der Nanopartikelsonde unter Bedingungen, die für ein Binden der Detektor-Aptamere an das Zielanalyts wirksam sind; und
c) Detektieren, ob die Detektor-Aptamere an das Zielanalyt binden,
wobei die Aptamere mit einer Oberflächendichte auf den Nanopartikeln vorhanden sind, die in einem Bereich von 8,9 x 10¹¹ bis 6,4 x 10¹² Aptameren/cm² liegt.

2. Das Verfahren nach Anspruch 1, wobei das Verfahren weiterhin in Schritt a) umfasst:
a) Bereitstellen eines Substrats, das zumindest eine Art von Capture-Sonde aufweist, welche an das Substrat gebunden ist, wobei die Capture-Sonde an eine zweite Bindungsstelle eines spezifischen Zielanalyts binden kann; und weiterhin in Schritt b) umfasst:
In Kontakt bringen der Probe mit dem Substrat unter Bedingungen, die für ein Binden der Capture-Sonde an die zweite Bindungsstelle des Zielanalyts wirksam sind, um einen Komplex aus Zielanalyt gebunden an die Sonde und das Substrat zu bilden; und in Schritt c) Detektieren der Anwesenheit oder Abwesenheit des Komplexes, wobei die Anwesenheit oder Abwesenheit des Komplexes auf die Anwesenheit oder Abwesenheit des spezifischen Zielanalyts in dem selben hinweist.

3. Das Verfahren nach Anspruch 1, wobei das Verfahren in Schritt a) umfasst:
Aptamere, die fähig sind, an zwei oder mehr Bindungsstellen des Zielanalyts zu binden.

4. Das Verfahren nach Anspruch 1, wobei das Verfahren in Schritt a) umfasst:
zumindest zwei Arten von Nanopartikelsonden, die Detektions-Aptamere aufweisen, die daran gebunden sind, wobei jede Art von Aptamer in der Lage ist, an eine unterschiedliche Bindungsstelle des Zielanalyts zu binden;
und in Schritt b) umfasst:
In Kontakt bringen der Probe mit den zumindest zwei Arten von Nanopartikelsonden, die daran gebundene Aptamere aufweisen, unter Bedingungen, die wirksam sind, um ein Binden zwischen dem Zielanalyt und den an die Nanopartikel gebundenen Aptameren zu erlauben.

5. Das Verfahren nach Anspruch 1, wobei das Verfahren weiterhin in Schritt a) umfasst:
Bereitstellen von zumindest einer Art von Nanopartikeln, die daran gebundene Antikörper aufweisen, wobei die Antikörper in der Lage sind, an die zweite Bindungsstelle des Zielanalyts zu binden; und weiterhin in Schritt b) umfasst:
In Kontakt bringen der Probe mit der zumindest einen Art von Nanopartikeln, die daran gebundene Antikörper aufweisen, unter Bedingungen, die wirksam sind, ein Binden zwischen dem Zielanalyt und den an die Nanopartikeln gebundenen Antikörper zu erlauben;
und in Schritt c) umfasst:
Detektieren der Anwesenheit oder Abwesenheit des Komplexes, wobei die Anwesenheit oder Abwesenheit des Komplexes auf die Anwesenheit oder Abwesenheit des spezifischen Zielanalyts in demselben hindeutet.

6. Das Verfahren nach Anspruch 1, 2, 3, oder 4 wobei in Schritt a):
zumindest eine Art von Detektor-Aptamer, das einen Oligonukleotid-Schwanz aufweist, bereitgestellt wird, und ein zweites Oligonukleotid bereitgestellt wird, das eine Sequenz hat, die komplementär zu zumindest einem Teil der Sequenz des Oligonukleotid-Schwanzes ist, wobei das zweite Oligonukleotid eine optionale Markierung aufweist.

7. Das Verfahren nach Anspruch 2 oder 6, wobei die Capture-Sonde einen Antikörper oder ein Capture-Aptamer umfasst.

8. Das Verfahren nach einem der Ansprüche 2, 6 und 7, wobei die Bindungsstellen Epitope sind, an die eine spezifische Capture-Sonde bindet.

9. Das Verfahren nach einem der Ansprüche 2, 6, 7 und 8, wobei zwei oder mehrere Arten von Nanopartikeln oder Aptamer-Sonden bereitgestellt werden, wobei jede Art von Sonde ein daran gebundenes Detektor-Aptamer aufweist, welches in der Lage ist, an ein unterschiedliches Epitop auf demselben Zielanalyt, oder an unterschiedliche Analyten oder an beides zu binden.

10. Das Verfahren nach einem der Ansprüche 2, 6 und 7 bis 9, wobei
i) die Probe zuerst mit der Nanopartikel- oder Aptamer-Sonde in Kontakt gebracht wird, so dass ein in der Probe befindlicher Zielanalyt an die Detektor-Aptamere auf der Sonde bindet, und der an die Nanopartikel- oder Aptamer-Sonde gebundene Zielanalyt dann mit dem Substrat in Kontakt gebracht wird, so dass der Zielanalyt an die Capture-Sonde auf dem Substrat bindet;
ii) die Probe zuerst mit dem Substrat in Kontakt gebracht wird, so dass ein in der Probe befindlicher Zielanalyt an die Capture-Sonde bindet, und der an das Capture-Aptamer gebundene Zielanalyt dann mit der Nanopartikel- oder Aptamer-Probe in Kontakt gebracht wird, sodass der Zielanalyt an die Detektor-Aptamere auf den Nanopartikel-oder Aptamer-Sonden bindet; oder
iii) die Probe, die Nanopartikel- oder Aptemer-Sonde und die Capture-Sonde auf dem Substrat gleichzeitig in Kontakt gebracht werden.

11. Das Verfahren nach einem der Ansprüche 2, 6 und 7 bis 10, wobei der gefangene Ziel-Nanopartikel oder -aptamer Sonden-Komplex mittels photonischen, elektronischen, akustischen, opto-akustischen, Schwerkrafts-, elektrochemischen, elektrooptischen, massenspektrometrischen, enzymatischen, chemischen, biochemischen oder physikalischen Mitteln detektiert wird.

12. Das Verfahren nach einem der Ansprüche 1 bis 5, wobei die Detektion mittels photonischer, elektronischer, akustischer, opto-akustischer, Schwerkrafts-, elektrochemischer, elektrooptischex, massenspektrometrischer, enzymatischer, chemischer, biochemischer oder physikalischer Mittel durchgeführt wird.

13. Das Verfahren nach einem der Ansprüche 2, 7 bis 10 und 12, wobei die Nanopartikel aus einem Edelmetall hergestellt sind, vorzugsweise aus Gold oder Silber, bevorzugter aus Gold.

14. Das Verfahren nach einem der Ansprüche 2, 6 und 7 bis 11, wobei das Substrat
α) ein magnetisches Kügelchen ist;
β) eine plane Oberfläche aufweist; oder
γ) aus Glas, Quarz, Keramik oder Kunststoff hergestellt ist.

15. Das Verfahren nach einem der Ansprüche 1 bis 6 und 7 bis 13, wobei das Detektieren umfasst:
1) In Kontakt bringen des Substrats mit einer Silberfärbung;
2) Detektieren des durch das Nanopartikel gestreuten Lichts; oder
3) Beobachtung mit einem optischen Scanner.

16. Das Verfahren nach Anspruch 15, wobei der Scanner mit einem Computer verbunden ist, auf den eine Software geladen wurde, die in der Lage ist, Graustufenmessungen zu berechnen, und wobei die Graustufenmessungen berechnet werden, um ein quantitatives Maß des detektierten Analyts bereitzustellen.

17. Das Verfahren nach einem der Ansprüche 2, 6, 7 bis 11, 15 und 16, wobei das Substrat adressierbar ist.

18. Das Verfahren nach Anspruch 17, wobei eine Vielzahl von Capture-Sonden in einer Punktmatrix an das Substrat angebracht ist, wobei jede Capture-Sonde ein unterschiedliches Zielanalyt erkennen kann.

19. Das Verfahren nach Anspruch 18, wobei jeder Punkt einer Capture-Sonde zwischen zwei Elektroden lokalisiert ist, die optionale Markierung der Aptamer-Sonde a) (ein) Nanopartikel ist/sind, das/die aus einem Material bestehen, das ein elektrischer Leiter ist, und Schritt d) die Detektion einer Veränderung in der Leitfähigkeit umfasst.

20. Das Verfahren nach Anspruch 19, wobei die Elektroden aus Gold hergestellt sind und die Nanopartikel aus Gold hergestellt sind.

21. Das Verfahren nach Anspruch 19, wobei das Substrat mit einer Silberfärbung in Kontakt gebracht wird, um eine Veränderung der Leitfähigkeit herbei zu führen.

22. Eine Nanopartikel-Aptamer-Konjugat-Sonde umfassend:
a) Nanopartikel; und
b) zumindest eine Art eines Aptamers, wobei die Aptamere mit einer Oberflächendichte auf den Nanopartikeln vorhanden sind, die in einem Bereich von 8,9 x 10¹¹ bis 6,4 x 10¹² Aptameren/ cm² liegt.

23. Die Sonde nach Anspruch 22, umfassend zumindest zwei Arten von Aptameren.

24. Die Sonde nach einem der Ansprüche 22 bis 23, wobei das Nanopartikel weiterhin zusätzlich zu den Aptameren daran angebrachte Oligonukleotide umfasst.

25. Die Sonde nach Anspruch 24, wobei die Oligonukleotide Polyadenosin-Oligonukleotide sind, vorzugsweise A₁₀ oder A₂₀.

26. Die Sonde nach einem der Ansprüche 22 bis 25, wobei die Aptamere Phosphorothioat-oder Phosphorodithioatgruppen umfassen.

27. Die Sonde nach einem der Ansprüche 22 bis 26, wobei die Nanopartikel metallische Nanopartikel oder Nanopartikel aus Halbleitern sind.

28. Die Sonde nach Anspruch 27, wobei die Nanopartikel aus einem Edelmetall hergestellt sind, vorzugsweise aus Gold.

29. Kit zur Detektion eines oder mehrerer Analyten in einer Probe, das Kit umfassend die Aptamer-Detektion-Sonden gemäß einem der Ansprüche 22 bis 28 und ein optionales Substrat.

30. Kit gemäß Anspruch 29, wobei das Substrat mit zumindest einer Capture-Sonde für ein spezifisches Analyt in einer Matrix angeordnet ist.

## Revendications

1. Procédé de détection d'au moins un type d'analyte cible dans un échantillon, l'analyte cible ayant au moins deux sites de liaison, le procédé comprenant les étapes consistant à :
a) fournir au moins un type de sonde de nanoparticule comprenant des aptamères de détection, où les aptamères de détection sur chaque type de sonde ont une configuration qui peut se lier à un premier site de liaison d'un type spécifique d'analyte cible ;
b) mettre en contact l'échantillon avec la sonde de nanoparticule dans des conditions qui sont efficaces pour la liaison des aptamères de détection à l'analyte cible ; et
c) détecter si les aptamères de détection se lient à l'analyte cible,
où les aptamères sont présents sur les nanoparticules à une densité de surface comprise entre 8,9 x 10¹¹ et 6,4 x 10¹² aptamères/cm².

2. Procédé selon la revendication 1, **caractérisé en ce qu'**il comprend en outre dans l'étape a) la fourniture d'un substrat ayant au moins un type de sonde de capture liée à celui-ci, où la sonde de capture peut se lier à un second site de liaison d'un analyte cible spécifique ;
et comprenant en outre dans l'étape b) la mise en contact de l'échantillon avec le substrat dans des conditions qui sont efficaces pour la liaison de la sonde de capture au second site de liaison de l'analyte cible pour former un complexe d'analyte cible lié à ladite sonde et audit substrat ; et
la détection dans l'étape c) de la présence ou de l'absence du complexe, où la présence ou l'absence du complexe est indicative de la présence ou de l'absence de l'analyte cible spécifique dans celui-ci.

3. Procédé selon la revendication 1, **caractérisé en ce qu'**il comprend dans l'étape a) des aptamères pouvant se lier à deux sites de liaison ou plus de l'analyte cible.

4. Procédé selon la revendication 1, **caractérisé en ce qu'**il comprend dans l'étape a) au moins deux types de sondes de nanoparticule ayant des aptamères de détection liés à ceux-ci, chaque type d'aptamère pouvant se lier à un site de liaison différent de l'analyte cible ; et dans l'étape b) la mise en contact de l'échantillon et des au moins deux types de sondes de nanoparticule ayant des aptamères liés à celles-ci dans des conditions efficaces pour permettre la liaison entre l'analyte cible et les aptamères liés aux nanoparticules.

5. Procédé selon la revendication 1, **caractérisé en ce qu'**il comprend en outre dans l'étape a) la fourniture d'au moins un type de nanoparticules ayant des anticorps liés à celles-ci, les anticorps pouvant se lier à un second site de liaison de l'analyte cible ;
et comprenant en outre dans l'étape b) la mise en contact dudit échantillon et du au moins un type de nanoparticules ayant des anticorps liés à celles-ci dans des conditions efficaces pour permettre la liaison entre l'analyte cible et
les anticorps liés aux nanoparticules ; et
la détection dans l'étape c) de la présence ou de l'absence du complexe, où la présence ou l'absence du complexe est indicative de la présence ou de l'absence de l'analyte cible spécifique dans celui-ci.

6. Procédé selon la revendication 1, 2, 3 ou 4, dans lequel dans l'étape a), au moins un type d'aptamère de détection ayant une queue d'oligonucléotide est prévu, et un second oligonucléotide ayant une séquence qui est complémentaire à au moins une partie d'une séquence de la queue d'oligonucléotide, ledit second oligonucléotide ayant une étiquette facultative, est prévu.

7. Procédé selon la revendication 2 ou 6, dans lequel la sonde de capture comprend un anticorps ou un aptamère de capture.

8. Procédé selon l'une quelconque des revendications 2, 6 et 7, dans lequel les sites de liaison sont des épitopes auxquels se lie une sonde de capture spécifique.

9. Procédé selon l'une quelconque des revendications 2, 6, 7 et 8, dans lequel deux types ou plus de sondes de nanoparticule ou d'aptamère sont prévues, chaque type de sonde ayant des aptamères de détection liés à celle-ci qui peuvent se lier à un épitope différent sur le même analyte cible ou sur des analytes cibles différents, ou sur les deux.

10. Procédé selon l'une quelconque des revendications 2, 6 et 7 à 9, dans lequel
i) l'échantillon est d'abord mis en contact avec la sonde de nanoparticule ou d'aptamère de sorte qu'un analyte cible présent dans l'échantillon se lie aux aptamères de détection sur la sonde, et l'analyte cible lié à la sonde de nanoparticule ou d'aptamère est ensuite mis en contact avec le substrat de sorte que l'analyte cible se lie à la sonde de capture sur le substrat ;
ii) l'échantillon est d'abord mis en contact avec le substrat de sorte qu'un analyte cible présent dans l'échantillon se lie à une sonde de capture, et l'analyte cible lié à l'aptamère de capture est ensuite mis en contact avec la sonde de nanoparticule ou d'aptamère de sorte que l'analyte cible se lie aux aptamères de détection sur la sonde de nanoparticule ou d'aptamère ; ou
iii) l'échantillon, la sonde de nanoparticule ou d'aptamère et la sonde de capture sur le substrat sont mis en contact simultanément.

11. Procédé selon l'une quelconque des revendications 2, 6 et 7 à 10, dans lequel le complexe de sonde d'aptamère ou de nanoparticule cible capturé est détecté par un moyen photonique, électronique, acoustique, optoacoustique, de gravité, électrochimique, électrooptique, de spectrométrie de masse, enzymatique, chimique, biochimique ou physique.

12. Procédé selon l'une quelconque des revendications 1 à 5, dans lequel la détection s'effectue par un moyen photonique, électronique, acoustique, optoacoustique, de gravité, électrochimique, électrooptique, de spectrométrie de masse, enzymatique, chimique, biochimique ou physique.

13. Procédé selon l'une quelconque des revendications 2, 7 à 10 et 12, dans lequel les nanoparticules sont constituées à partir d'un métal noble, de préférence d'or ou d'argent, de manière davantage préférée d'or.

14. Procédé selon l'une quelconque des revendications 2, 6, et 7 à 11, dans lequel le substrat
α) est une bille magnétique ;
β) a une surface planaire ; ou
γ) est constitué à partir de verre, de quartz, de céramique
ou de plastique.

15. Procédé selon l'une quelconque des revendications 1 à 6 et 7 à 13, dans lequel la détection comprend
1) la mise en contact du substrat avec un colorant à l'argent ;
2) la détection de la lumière diffusée par la nanoparticule ; ou
3) l'observation avec un scanner optique.

16. Procédé selon la revendication 15, dans lequel le scanner est lié à un ordinateur sur lequel est chargé un logiciel pouvant calculer les mesures d'échelle de gris, les mesures d'échelle de gris étant calculées pour donner une mesure quantitative de la quantité d'analyte détecté.

17. Procédé selon l'une quelconque des revendications 2, 6, 7 à 11, 15 et 16, dans lequel le substrat est adressable.

18. Procédé selon la revendication 17, dans lequel une pluralité de sondes de capture, dont chacune peut reconnaître un analyte cible différent, est attachée au substrat dans un réseau de points.

19. Procédé selon la revendication 18, dans lequel chaque point de sondes de capture est situé entre deux électrodes, l'étiquette facultative sur la sonde d'aptamère dans l'étape a) est la ou les nanoparticules constituées à partir d'un matériau qui est un conducteur d'électricité, et l'étape d) comprend la détection d'un changement de conductivité.

20. Procédé selon la revendication 19, dans lequel les électrodes sont constituées à partir d'or et les nanoparticules sont constituées à partir d'or.

21. Procédé selon la revendication 19, dans lequel le substrat est mis en contact avec un colorant à l'argent pour produire le changement de conductivité.

22. Sonde de conjugué nanoparticule-aptamère comprenant :
a) des nanoparticules ; et
b) au moins un type d'aptamère, les aptamères étant présents sur les nanoparticules à une densité de surface comprise entre 8,9 x 10¹¹ et 6,4 x 10¹² aptamères/cm².

23. Sonde selon la revendication 22, comprenant au moins deux types d'aptamères.

24. Sonde selon l'une quelconque des revendications 22 à 23, dans laquelle la nanoparticule comprend en outre les oligonucléotides attachés à celle-ci en plus des aptamères.

25. Sonde selon la revendication 24, dans laquelle les oligonucléotides sont des oligonucléotides de polyadénosine, de préférence A₁₀ ou A₂₀.

26. Sonde selon l'une quelconque des revendications 22 à 25, dans laquelle les aptamères comprennent des fractions phosphorothioate ou phosphorodithioate.

27. Sonde selon l'une quelconque des revendications 22 à 26, dans laquelle les nanoparticules sont des nanoparticules métalliques ou des nanoparticules semi-conductrices.

28. Sonde selon la revendication 27, dans laquelle les nanoparticules sont constituées à partir d'un métal noble, de préférence d'or.

29. Kit de détection d'un ou plusieurs analytes dans un échantillon, ledit kit comprenant les sondes de détection d'aptamère selon l'une quelconque des revendications 22 à 28 et un substrat facultatif.

30. Kit selon la revendication 29, dans lequel le substrat est disposé avec au moins une sonde de capture pour un analyte spécifique.
